(19) Europäisches Patentamt
European Patent Office
Office européen des brevets

(11) Veröffentlichungsnummer: **0 083 305**
**B1**

(12) **EUROPÄISCHE PATENTSCHRIFT**

(45) Veröffentlichungstag der Patentschrift: **10.07.85**

(51) Int. Cl.⁴: **C 07 K 7/64**, A 61 K 37/02

(21) Anmeldenummer: **82810552.8**

(22) Anmeldetag: **17.12.82**

(54) **Cyclische Octapeptide und pharmazeutische Präparate davon, sowie Verfahren zur Herstellung derselben und ihre Anwendung.**

(30) Priorität: **24.12.81 CH 8282/81**

(43) Veröffentlichungstag der Anmeldung: **06.07.83 Patentblatt 83/27**

(45) Bekanntmachung des Hinweises auf die Patenterteilung: **10.07.85 Patentblatt 85/28**

(84) Benannte Vertragsstaaten: **AT BE CH DE FR IT LI LU NL SE**

(56) Entgegenhaltungen:
**EP-A-0 030 920**
**EP-A-0 031 303**

(73) Patentinhaber: **CIBA-GEIGY AG, Postfach, CH-4002 Basel (CH)**

(72) Erfinder: **Kamber, Bruno, Dr., Sonnenweg 9, CH-4144 Arlesheim (CH)**

**EP 0 083 305 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Cyclopeptide vom Somatostatin-Typ und ihre Herstellungsverfahren sowie pharmazeutische Präparate enthaltend diese Verbindungen und Verwendung dieser Verbindungen bzw. Präparate zu therapeutischen Zwecken.

Die Erfindung betrifft insbesondere Cyclopeptide, welche die wesentlichsten Merkmale des Somatostatins, wie die Teilsequenz der Aminosäuren 6—11, nicht aber die beiden schwefelhaltigen Cysteinreste enthalten. Die erfindungsgemäßen Somatostatin-analogen Cyclopeptide umfassen cyclische Octapeptide der Formel

Aaa($Ac_A$)-Phe-Phe-trp-Lys($Ac_B$)-Thr-Phe-Gaba (I)

5 6 7 8 9 10 11 12

worin

Aaa der Rest einer geradkettigen $\alpha,\omega$-Diaminoalkansäure mit 4—7 C-Atomen

trp der Rest L-Trp oder insbesondere D-Trp, oder ein davon abgeleiteter Rest, der im Indolkern ein Halogen trägt,

$Ac_A$ ein an der $\omega$-Aminogruppe befindlicher Acylrest Ac einer gegebenenfalls substituierten Carbonsäure, die Amidinogruppe oder Wasserstoff, und

$Ac_B$ ein an der $\varepsilon$-Aminogruppe befindlicher Acylrest $Ac^1$ einer Aminosäure oder eines Oligopeptids, oder vorzugsweise Wasserstoff

ist, sowie Salze und Komplexe davon.

Somatostatin, ein cyclisches Tetradecapeptid der Formel

H-Ala-Gly-Cys-Lys-Asn-Phe-Phe-Trp-Lys-Thr-Phe-Thr-Ser-Cys-OH

1 2 3 4 5 6 7 8 9 10 11 12 13 14

[Science 179, 77 (1973)] hemmt bekanntlich die hypophysengesteuerte Sekretion des Wachstumshormons (Somatotropin). Außerdem hemmt es die sekretorische Aktivität des endocrinen Pancreas, wie die Sekretion von Insulin und Glucagon. Diese wertvollen Eigenschaften können beim Somatostatin selber nicht zur vollen praktischen Anwendung gelangen, da diese Verbindung eine zu kurze Wirkungsdauer aufweist. Zudem ist es oft von Vorteil, wenn der Wirkstoff seine hemmende Wirkung hauptsächlich auf eine der beiden Drüsen ausübt, wogegen die andere Drüse möglichst wenig beeinflußt werden sollte. (In den meisten Fällen ist die Hemmung der hypophysären Sekretion, d. h. die der Wachstumshormon-Ausschüttung, weniger erwünscht.) Deshalb wird angestrebt, durch Modifizierung der Grundsequenz, insbesondere durch Auslassen einzelner ursprünglicher Aminosäuren und/oder deren Austausch gegen andere, oft auch »unnatürliche« Aminosäuren, eine Dissociation der Hemmwirkungen sowie eine möglichst lange Wirkungsdauer zu erlangen. So wurde z. B. gefunden, daß besonders vorteilhafte physiologische Eigenschaften dieser Art bei Cyclopeptiden vom Typ

Asn-Phe-Phe-[D-Trp]-Lys-Thr-Phe-$[Gaba]_p$ (M)

5 6 7 8 9 10 11 12

worin Gaba den Rest der $\gamma$-Aminobuttersäure und p die Zahl 0, 1 oder 2 darstellt, und weiteren nahe verwandten Verbindungen auftreten, vgl. unser US-Patent 4 238 481. Als ein typisches Merkmal dieser Verbindungen kommt das Vorhandensein einer geraden Kohlenstoffkette vor, welche die ursprüngliche Gruppierung

$$-CH_2-S-S-CH_2-$$

im Cysteinrest von Somatostatin simulieren soll.

Überraschenderweise wurde nun festgestellt, daß durch eine ganz ungewöhnliche Modifizierung der Grundstruktur dieses einfachen Wirkstoffes der Formel M, und zwar durch den bei Somatostatin-Analogen bisher nicht angewendeten Austausch des Asparagin-Restes in der 5-Stellung gegen den Rest einer $\alpha$-Aminosäure, die am endständigen C-Atom eine gegebenenfalls modifizierte Aminogruppe trägt, ein Analog entsteht, in welchem die ursprüngliche Aktivität der Grundstruktur M nicht nur erhalten bleibt, sondern oft im oben diskutierten Sinne, insbesondere in bezug auf die Wirkungsintensität

und Wirkungsdauer, weiter gesteigert und vertieft wird. Ein solches Resultat überrascht um so mehr, als man allgemein voraussetzt, daß ein Substituent vom Typ der endständigen Aminogruppe im Rest der zugetretenen Austausch-Aminosäure die biologische Wirkung des ursprünglichen Wirkstoffes durch seinen vorwiegend basischen Charakter aufheben oder in eine ganz andere Richtung lenken kann. Beispielsweise wurde bei der Bestimmung der Insulin- und Glucagon-Ausschüttung am isolierten, perfundierten, mit Arginin stimulierten Ratten-Pankreas [Methode gemäß B. Petrack, A. J. Czernik, W. Itterly, J. Ansell und H. Chertock: Biochem. and Biophys. Res. Commun. 73, 934—939 (1976)] gefunden, daß das [$Lys^5$,D-$Trp^8$,$Gaba^{12}$]-cyclo-somatostatin(5—12)-octapeptid eine 5—10fache Hemmwirkung auf die Insulin- und Glucagon-Sekretion, verglichen mit [D-$Trp^8$,$Gaba^{12}$]-cyclo-somatostatin(5—12)-octapeptid der oben angegebenen Formel M (p = 1), ausübt.

Ein unter dem Symbol Aaa in der Formel I angegebener Rest einer $\alpha,\omega$-Diaminoalkansäure leitet sich vom Grundgerüst der Buttersäure oder Önanthsäure (Heptansäure), vorzugsweise aber der Valeriansäure oder vor allem der Capronsäure (Hexansäure) ab und ist in den peptidischen Ring über die $\alpha$-Aminogruppe eingebaut. Diese hat vorzugsweise dieselbe sterische Anordnung wie die in der Natur vorkommenden $\alpha$-Aminosäuren der L-Reihe. Als besonders bevorzugte Reste mit freier endständiger $\omega$-Aminogruppe ($Ac_A$ = Wasserstoff), kommen diejenigen des L-Ornithins und vor allem des L-Lysins, ferner auch des D-Lysins in Betracht; besonders hervorzuheben sind auch analoge Reste, worin die endständige $\omega$-Aminogruppe im Rahmen der Bedeutungen von $Ac_A$ funktionell abgewandelt ist, z. B. durch die Amidinogruppe $NH_2-C(=NH)-$ (wie vor allem im Arginin-Rest) oder durch einen Acylrest Ac, insbesondere einen von einer $\alpha$-Aminosäure oder einem Oligopeptid abgeleiteten Acylrest $Ac^1$, z. B. einen der weiter unten besonders hervorgehobenen, substituiert ist.

In den eingangs definierten Verbindungen der Formel I bedeutet trp vorzugsweise D-Trp, ferner auch einen L- oder D-Trp-Rest, der im Indolkern, insbesondere in der 5-Stellung, ein Halogenatom, wie Chlor oder insbesondere Fluor, trägt, z. B. insbesondere [D-(5 F)Trp], der sich von D-5-Fluortryptophan ableitet.

In den eingangs definierten Verbindungen der Formel I ist durch das Symbol Gaba die $\gamma$-Aminobuttersäure bezeichnet.

Falls in der Formel I das Symbol $Ac_A$ bzw. $Ac_B$ für Wasserstoff steht, bedeutet es, daß die endständige Aminogruppe des $Aaa^5$- bzw. $Lys^9$-Rests frei ist.

Die dem Acylrest Ac zugrunde liegende Carbonsäure ist z. B. eine Alkancarbonsäure, die vorzugsweise nicht mehr als 18 Kohlenstoffatome, wenn sie unsubstituiert ist, und vorzugsweise nicht mehr als 8 Kohlenstoffatome, wenn sie substituiert ist, besitzt. Die Substituenten sind einerseits Hydroxyl-, Mercapto-, Niederalkylmercapto-, wie Methylmercapto-, Guanidino-, Carboxyl- und Carboxamido-, sowie Aminogruppen, oder eine an 2 verschiedenen Kohlenstoffatomen gebundene Iminogruppe, andererseits mono- oder bicyclische Hydrocarbyl- oder Heterocyclylreste, wie insbesondere Phenyl, p-Hydroxyphenyl, 1- oder 2-Naphthyl-, 2-, 3- oder 4-Pyridyl, 2- oder 3-Indolyl, 2- oder 4-Imidazolyl, 2-, 4- oder 5-Thiazolyl, 2-Thienyl oder 2-Furyl. Die Säure kann einen oder mehrere gleichartige oder verschiedenartige Substituenten tragen, wobei die gesamte Anzahl der Kohlenstoffatome einschließlich der kohlenstoffhaltigen Substituenten vorzugsweise höchstens 18 C-Atome beträgt. Besonders bevorzugt sind Acylreste, abgeleitet von einfach verzweigten oder insbesondere von geradkettigen unsubstituierten Alkanmonocarbonsäuren, die höchstens 18, vorzugsweise höchstens 9 Kohlenstoffatome besitzen, wie von der Essig-, Propion-, Butter-, Isobutter-, Valerian-, Isovalerian-, Capron-, Önanth-, Undecan-, Laurin-, Myristin-, Palmitin- und Stearinsäure.

Bevorzugt unter den Acylresten Ac sind die mit dem Symbol $Ac^1$ bezeichneten Reste von $\alpha$-Aminosäuren, insbesondere solchen, die sich von Alkancarbonsäuren mit höchstens 7 C-Atomen ableiten und die neben der Aminogruppe auch die oben angegebenen Substituenten tragen können. Darunter sind solche Reste besonders bevorzugt, die sich von in der Natur, insbesondere als Peptid-Bausteine, vorkommenden $\alpha$-Aminosäuren der L-Reihe und deren nahe verwandten Analogen, wie insbesondere den Enantiomeren der »unnatürlichen« D-Reihe, ableiten. Unter den bevorzugten $\alpha$-Aminosäuren kommen beispielsweise die folgenden ganz besonders in Betracht: Glycin, Alanin, Valin, Leucin, Isoleucin, Phenylalanin, Asparaginsäure, Glutaminsäure, Arginin, Histidin und vor allem Lysin, ferner $\alpha$-Aminobuttersäure, Norvalin, Isovalin, Norleucin, Ornithin und Citrullin sowie auch Asparagin, Glutamin, Tyrosin, Tryptophan, Methionin, Threonin, Serin und ganz besonders auch Prolin und Hydroxyprolin (bei denen die $\alpha$-Aminogruppe mit dem Alkylrest zu einem Ring zusammengeschlossen ist). — Als Reste $Ac^1$ kommen auch Oligopeptid-, insbesondere Di- und Tri-peptid-Reste in Betracht. Diese sind vorzugsweise aus den in der Natur vorkommenden $\alpha$-Aminosäuren (vor allem den oben besonders hervorgehobenen, wie insbesondere Lysin oder Prolin) zusammengesetzt. Vorzugsweise wiederholt sich in der Oligopeptidkette mehrmals der Rest einer einzigen Aminosäure, wie z. B. im H-Lys-Lys-Lys- oder anderen analog gebauten dipeptidischen und tripeptidischen Acylresten.

Als bevorzugte Reste -Aaa($Ac^1$)- sind beispielsweise zu nennen:

| | | | | |
|---|---|---|---|---|
| H-Phe—┐ | H-His—┐ | H-Lys—┐ | H-(D-Lys)—┐ | H-Pro—┐ |
| -Lys- | -Lys- | -Lys- | -Lys- | -Lys- |

H-Lys-Lys┐ H-Lys-Lys-Lys┐
-Lys- und -Lys-

ferner auch analog gebaute Reste, worin anstelle eines oder mehrere Lysin-Reste (gleichwohl, ob als der Rest Aaa der Austausch-Aminosäure und/oder ein Bestandteil des Acylrests $Ac^1$) der Ornithin-Rest steht. (Die oben angewendete Symbolik besagt im Einklang mit den geläufigen nomenklaturischen Regeln, daß der untere Rest, d. h. -Lys-, in der Seitenkette, d. h. an der $\varepsilon$-Aminogruppe, durch den oberen Acylrest, z. B. H-Pro- oder H-Lys-Lys-Lys-, substituiert ist).

Besonders bevorzugte erfindungsgemäße Cyclooctapeptide der Formel I sind z. B. die folgenden:

[Lys$^5$,D-Trp$^8$,Gaba$^{12}$]-cyclo-somatostatin(5—12)octapeptid der Formel

Lys-Phe-Phe-(D-Trp)-Lys-Thr-Phe-Gaba

[Orn$^5$,D-Trp$^8$,Gaba$^{12}$]-cyclo-somatostatin(5—12)octapeptid der Formel

Orn-Phe-Phe-(D-Trp)-Lys-Thr-Phe-Gaba

[N$^{\varepsilon}$-(Pro)-Lys$^5$,D-Trp$^8$,Gaba$^{12}$]-cyclo-somatostatin(5—12)octapeptid der Formel

H-Pro┐
Lys-Phe-Phe-(D-Trp)-Lys-Thr-Phe-Gaba

[N$^{\varepsilon}$-(Lys)-Lys$^5$,D-Trp$^8$,Gaba$^{12}$]-cyclo-somatostatin(5—12)octapeptid der Formel

H-Lys┐
Lys-Phe-Phe-(D-Trp)-Lys-Thr-Phe-Gaba

[N$^{\varepsilon}$-(Phe)-Lys$^5$,D-Trp$^8$,Gaba$^{12}$]-cyclo-somatostatin(5—12)octapeptid der Formel

H-Phe┐
Lys-Phe-Phe-(D-Trp)-Lys-Thr-Phe-Gaba

und
[N$^{\varepsilon}$-(Lys-Lys-Lys)-Lys$^5$,D-Trp$^8$,Gaba$^{12}$]-cyclo-somatostatin(5—12)octapeptid der Formel

H-Lys-Lys-Lys┐
Lys-Phe-Phe-(D-Trp)-Lys-Thr-Phe-Gaba

sowie auch
[N$^{\delta}$-(Pro)-Orn$^5$,D-Trp$^8$,Gaba$^{12}$]-cyclo-somatostatin(5—12)octapeptid,
[N$^{\delta}$-(Orn)-Orn$^5$,D-Trp$^8$,Gaba$^{12}$]-cyclo-somatostatin(5—12)octapeptid,
[N$^{\delta}$-(His)-Orn$^5$,D-Trp$^8$,Gaba$^{12}$]-cyclo-somatostatin(5—12)octapeptid oder
[Lys$^5$,D-(5 F)Trp$^8$,Gaba$^{12}$]-cyclo-somatostatin(5—12)octapeptid.

Diejenigen der oben allgemein oder als bevorzugt charakterisierten Cyclopeptide der Formel I, die eine freie Aminogruppe oder Amidinogruppe enthalten, können auch in Form von Säureadditionssalzen vorliegen. Als Säureadditionssalze kommen insbesondere physiologisch verträgliche Salze mit üblichen therapeutisch anwendbaren Säuren in Betracht; von anorganischen Säuren sind die Halogenwasserstoffsäuren (wie die Chlorwasserstoffsäure), aber auch Schwefelsäure und Phosphor- bzw. Pyrophosphorsäure zu nennen, von organischen Säuren sind es in erster Linie die Sulfonsäuren (wie die Benzol- oder p-Toluol-sulfonsäure oder Niederalkansulfonsäuren, wie Methansulfonsäure) sowie auch Carbonsäuren, wie Essigsäure, Milchsäure, Palmitin- und Stearinsäure, Äpfelsäure, Weinsäure, Ascorbinsäure und Zitronensäure. Diejenigen der durch die Formel I charakterisierten Cyclopeptide, die in den

Resten $Ac_A$ und/oder $Ac_B$ eine freie Carboxylgruppe enthalten, die dem ganzen Cyclopeptid sauren Charakter erteilt, können auch als Salze vorliegen, z. B. Natrium-, Kalium-, Calcium- oder Magnesiumsalze, oder auch Ammoniumsalze abgeleitet von Ammoniak oder einer physiologisch verträglichen organischen stickstoffhaltigen Base.

Diejenigen der Verbindungen der Formel I, die sowohl eine freie Carboxylgruppe wie auch eine freie Aminogruppe (bzw. Amidinogruppe) enthalten, können auch als innere Salze vorliegen.

Die erfindungsgemäßen Peptide der Formel I können auch als Komplexe vorliegen. Unter Komplexen sind die in ihrer Struktur noch nicht völlig abgeklärten Verbindungen zu verstehen, die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z. B. anorganische Verbindungen, die sich von Metallen wie Calcium, Magnesium, Aluminium, Cobalt und insbesondere Zink ableiten, vor allem schwerlösliche Salze, wie Phosphate, Pyrophosphate und Polyphosphate, sowie Hydroxide dieser Metalle, ferner Alkalimetallpolyphosphate, z. B. »Calgon® N«, »Calgon® 322«, »Calgon® 188« oder »Polyron® 12«. Organische Stoffe, die eine Verlängerung der Wirkung hervorrufen, sind beispielsweise nicht-antigene Gelatine-Arten (z. B. Polyoxygelatine), Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z. B. Protamin oder Polyglutaminsäure.

Wenn nicht anders angegeben, beziehen sich die Kurzbezeichnungen der Aminosäurereste auf Reste der $\alpha$-Aminosäuren der in der Natur vorkommenden L-Reihe. Zur Verdeutlichung wird in Formeln der Rest von D-Tryptophan als -(D-Trp)- bezeichnet.

Wenn nicht anders angegeben, bezeichnet der Ausdruck »nieder«, wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemäßen Cyclopeptide weisen eine physiologische Wirkung auf, die im Grundcharakter der Wirkung des Somatostatins ähnlich ist. Sie können deshalb in ähnlichen therapeutischen Indikationen wie dieses, z. B. insbesondere zur Behandlung von Funktionsstörungen, in denen die Sekretion des Wachstumshormons oder des Glucagons übernormal hoch ist, wie bei Acromegalie oder Diabetes, mit Vorteil angewendet werden. Indem sie überdies noch Blutverluste im gastro-intestinalen Trakt hemmen, können sie auch in diesem Indikationsbereich mit Erfolg eingesetzt werden.

Die erfindungsgemäßen Cyclopeptide werden unter Anwendung von konventionellen, an sich bekannten Herstellungsverfahren der Peptidchemie erhalten.

So werden sie beispielsweise hergestellt, indem man ein dem Cyclopeptid entsprechende lineares Peptid cyclisiert, d. h. ein solches, das dieselben Aminosäuren in gleicher Reihenfolge wie das erfindungsgemäße cyclische Peptid hat, wobei aber eine amidische Bindung zwischen zwei beliebigen benachbarten ringbildenden Aminosäuren unterbrochen ist und durch entsprechende endständige funktionelle Gruppen, d. h. eine Carboxy- und eine Aminogruppe, die auch in einer aktivierten Form vorliegen können, ersetzt ist.

Die erfindunggemäßen Verbindungen werden durch Cyclisierung insbesondere so hergestellt, daß man ein entsprechendes lineares Peptid der Formel

$$H-[I_a]-V \qquad (II)$$

worin $I_a$ einen der Formel I entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazingruppe $-NH-NH_2$ steht, cyclisiert, wobei gegebenenfalls vorhandene, an der Cyclisierungsreaktion nicht beteiligte Amino-, Carboxyl- und Hydroxylgruppen nach Bedarf vorübergehend in geschützter Form vorliegen und anschließend freigesetzt werden, und, wenn erwünscht, in einem erhaltenen Cyclopeptid vorhandene freie Aminogruppen, nötigenfalls unter vorübergehendem Schutz vorhandener Hydroxylgruppen und übriger Aminogruppen, acyliert.

Unter den linearen Peptiden der Formel II sind diejenigen bevorzugt, worin der Rest Gaba oder insbesondere der Rest Aaa als eine endständige Aminosäure im Rest $[I_a]$ steht. Ein besonders bevorzugter Ausgangsstoff ist durch die Formel

$$H\text{-}Aaa(Ac_a)\text{-}Phe\text{-}Phe\text{-}trp\text{-}Lys(Ac_b)\text{-}Thr\text{-}Phe\text{-}Gaba\text{-}V \qquad (IIa)$$

charakterisiert, worin $Ac_a$ den oben charakterisierten Acylrest Ac oder eine $\omega$-Aminoschutzgruppe $X_o$, und $Ac_b$ den oben charakterisierten Acylrest einer $\alpha$-Aminosäure bzw. eines Oligopeptids $Ac^1$ oder eine $\omega$-Aminoschutzgruppe $X_o$ bedeutet, Aaa und trp die eingangs genannten und V die unmittelbar oben angegebenen Bedeutungen haben. (Der vorübergehende Schutz vorhandener Carboxyl-, Hydroxyl- und Aminogruppen während der Cyclisierung betrifft auch die entsprechenden Gruppen in den Resten Ac und $Ac^1$).

Eine durch das Symbol V dargestellte funktionelle Gruppe ergänzt die Carbonylgruppe des C-terminalen Aminosäure-Reste und bildet zusammen mit ihr eine freie Carboxylgruppe, eine aktivierte Estergruppe, beziehungsweise die Carbazolylgruppe.

Die Aktivierungsgruppe, durch welche die Hydroxylgruppe abgewandelt ist, ist insbesondere eine solche, die den aktivierten Ester von N-Hydroxysuccinimid, 1-Hydroxybenzotriazol, N,N'-Dicyclohexylisoharnstoff, 2,4,5-Trichlorphenol, 2-Nitrophenol, 4-Nitrophenol, Pentachlorphenol oder Pentafluorphenol bildet, aber auch eine andere aus der Peptidchemie bekannte Aktivierungsgruppe dieser Art, vgl. Houben-Weyl, Band 15/II.

Die erfindungsgemäße Cyclisierung der linearen Peptide der Formel II bzw. IIa erfolgt in an sich bekannter Weise mittels üblicher, zur Ausbildung der Amid-Bindung gebräuchlicher Kupplungsmethoden, wobei aber die peptidischen Ausgangsstoffe in einer sehr niedrigen Konzentration eingesetzt werden, um den Verlauf der Kupplung zugunsten der intramolekularen Cyclisierung auf Kosten der intermolekularen Polykondensation zu verschieben.

Die linearen Peptide werden mit Vorteil in einer etwa $1 \cdot 10^{-4}$-molaren bis etwa $1 \cdot 10^{-2}$-molaren, vorzugsweise einer etwa $1 \cdot 10^{-3}$-molaren Konzentration eingesetzt, die einer Gewicht/Volumen-Konzentration von etwa 0,01 bis 1,0%, vorzugsweise 0,1 % entspricht. Eine entsprechende Verdünnung kann im Reaktionsgemisch vom Anfang an eingestellt werden, oder aber durch langsames Eintropfen des Ausgangsstoffes und gegebenenfalls der übrigen Reagentien in das Reaktionsgemisch fortlaufend hergestellt werden.

Vorzugsweise erfolgt die Cyclisierung, indem man bei einer oben angegebenen Anfangskonzentration entweder a) einen Ausgangsstoff der Formel II, worin V eine freie Hydroxylgruppe bedeutet, unter vorübergehendem Schutz vorhandener übriger Amino-, Carboxyl-, sowie Hydroxylgruppen mit einem Carbodiimid, gegebenenfalls in Anwesenheit einer Aktivester-bildenden Komponente, behandelt oder b) einen Ausgangsstoff der Formel II, worin V eine zum aktivierten Ester abgewandelte Hydroxylgruppe darstellt und die endständige $\alpha$-Aminogruppe in protonierter Form vorliegt, wobei mindestens die vorhandenen an der Cyclisierung nicht beteiligten Aminogruppen und Carboxylgruppen geschützt sind, mit einer organischen Base umsetzt oder c) einen Ausgangsstoff der Formel II, worin V die Gruppe $-NHNH_2$ bedeutet, wobei mindestens die vorhandenen an der Cyclisierung nicht beteiligten Aminogruppen geschützt sind, zunächst unter sauren Bedingungen mit salpetriger Säure oder einem Niederalkylester davon behandelt und nacher bei einer oben erwähnten niedrigen Konzentration mit überschüssiger organischer Base cyclisiert.

Eine Carboxylgruppe wird in der weiter unten beschriebenen Weise durch eine Schutzgruppe W geschützt. Zum Schutz der Amino- und Hydroxylgruppen werden zweckmäßig die Gruppen $X_o$ und $X_a$ bzw. Y verwendet, wie sie weiter unten definiert sind.

Die Cyclisierung führt man in geeigneten Lösungsmitteln durch; beispielsweise sind Dioxan, Tetrahydrofuran, Acetonitril, Pyridin, Dimethylformamid, Dimethylacetamid, Dimethylsulfoxid, N-Methylpyrrolidon, Hexamethylphosphortriamid sowie auch Chloroform, Methylenchlorid und Äthylacetat und Gemische davon zu nennen.

Bei der Verfahrensvariante a) wird die Cyclisierung durch ein Carbodiimid, vorzugsweise N,N'-Dicyclohexylcarbodiimid, das man mit Vorteil im Überschuß anwendet, herbeigeführt; es ist anzunehmen, daß dabei der Ausgangsstoff der Formel II mit freier Carboxylgruppe primär in einen aktivierten Ester des Dicyclohexylisoharnstoffes (bzw. eines analogen Isoharnstoffes) übergeht und dieser in situ gebildete aktive Ester gleich weiterreagiert. Auf eine intermediäre Bildung eines aktiven Esters ist zweifellos bei Zugabe einer Aktivester-bildenden Komponente als Hilfsreagenz zu schließen; zu diesem Zwecke können in der Peptidchemie übliche Aktivester-bildende Komponenten dienen, wie insbesondere 2,4,5-Trichlorphenol, 2- oder 4-Nitrophenol, Pentachlor- und Pentafluorphenol, aber vor allem N-Hydroxyverbindungen, wovon als besonders vorteilhaft N-Hydroxysuccinimid, N-Hydroxypiperidin und vor allem 1-Hydroxybenzotriazol zu erwähnen sind. Die Arbeitstemperatur bei dieser Variante beträgt im allgemeinen 0—70°, vorzugsweise 35—55°.

Bei der Variante b), die mit fertigen Aktivestern, insbesondere den bereits hervorgehobenen, arbeitet, erfolgt die Cyclisierung spontan, als die endständige $\alpha$-Aminogruppe durch die organische Base entprotoniesiert wird. Die verwendeten Basen sind vorzugsweise quaternäre oder vor allem tertiäre Amine, z. B. Triäthylamin oder N-Äthylmorpholin. Vorzugsweise arbeitet man bei 10—30°, insbesondere bei Raumtemperatur.

Bei der Variante c) kann die erste Phase, d. h. die Bildung des Säureazids durch Behandlung mit salpetriger Säure oder einem Ester davon, mit Vorteil bei einer wesentlich höheren Konzentration der Ausgangsstoffe als die nachfolgende Cyclisierung erfolgen. Zweckmäßig arbeitet man mit etwa einem Äquivalent eines Niederalkylnitrits, wie Äthyl-, Isoamyl- und insbesondere tert-Butyl-nitrit, in salzsaurem Milieu bei Temperaturen von etwa —30 bis etwa —5°, vorzugsweise etwa —20°; ein geringer Überschuß an Nitrit ist zulässig. Danach wird die Lösung des gebildeten Azids nach der notwendigen Verdünnung bei einer Temperatur von 0 bis etwa 35° mittels einer überschüssigen organischen Base, z. B. einer der obengenannten, basisch gestellt und dadurch wie bei der Verfahrensvariante b) zur spontanen Cyclisierung gebracht.

Die engere Auswahl der Schutzgruppen richtet sich nach dem spezifischen Zweck, wobei man insbesondere bei mehreren zu schützenden funktionellen Gruppen, z. B. in den Resten $Ac_A$ und $Ac_B$, zweckmäßige Kombinationen wählen muß.

Als $\omega$-Amino-Schutzgruppe $X_o$ kann jede in der Peptid-Chemie übliche Amino-Schutzgruppe verwendet werden, wie sie in den entsprechenden Nachschlagewerken, z. B. in Houben-Weyl: Methoden der organischen Chemie; 4. Auflage, Band 15/I, E. Wünsch (Herausgeber): Synthese von Peptiden (Georg Thieme Verlag, Stuttgart; 1974) zusammenfassend beschrieben werden. So kann man z. B. die von schwefelhaltigen organischen Säuren, wie von organischen Sulfonsäuren, abgeleiteten Acylgruppen (wie p-Toluolsulfonyl sowie auch Benzolsulfonyl und o-Nitro-phenylsulfenyl) verwenden, vor allem aber Carbonsäure-Acylreste. Ein solcher als Schutzgruppe anzuwendender Acylrest unterscheidet sich vom oben charakterisierten Acylrest Ac grundsätzlich dadurch, daß er von der ihn tragenden $\omega$-Aminogruppe unter Freigabe der Aminogruppe selektiv abspaltbar ist, wobei die peptidische Grundstruktur unversehrt bleibt, wogegen ein Acylrest Ac von der $\omega$-Aminogruppe ohne gleichzeitige Beeinträchtigung der peptidischen Amid-Bindung nicht abzulösen ist.

Die durch das Symbol $X_o$ bezeichneten, als $\omega$-Aminoschutzgruppe verwendbaren Carbonsäure-Acylreste sind z. B. Formyl, Trifluoracetyl, oder Phthaloyl, aber insbesondere Reste, die sich von der Kohlensäure, und speziell von ihren Monoestern, ableiten. Unter diesen sind es vor allem acidolytisch abspaltbare Acylreste, wie in erster Linie die Reste vom Typ tert-Butoxycarbonyl, wie tert-Amyloxycarbonyl, Isopropyloxycarbonyl, Diisopropylmethoxycarbonyl, Allyloxycarbonyl, Cyclopentyloxycarbonyl, Cyclohexyloxycarbonyl, d-Isobornyloxycarbonyl und Adamantyloxycarbonyl, sowie auch bestimmte Aralkoxycarbonyl-Reste des 2-(p-Biphenylyl)-2-propyloxycarbonyl-Typs, die in der schweizerischen Patentschrift 509 266 beschrieben sind. Ferner gehören unter die Acylreste $X_o$ auch reduktiv oder basisch abspaltbare Acylreste, z. B. insbesondere die vom Benzyloxycarbonyl-Typ, wie Benzyloxycarbonyl selber und seine Derivate, die im aromatischen Teil durch Halogenatome, Nitrogruppen, Niederalkoxygruppen und/oder Niederalkylreste substituiert sind, wie p-Chlor- und p-Brombenzyloxycarbonyl, p-Nitrobenzyloxycarbonyl, p-Methoxybenzyloxycarbonyl, p-Tolyloxycarbonyl sowie insbesondere 4-Pyridylmethoxycarbonyl. Ein besonders vorteilhafter Acylrest $X_o$ ist ein Äthoxycarbonylrest, der in $\beta$-Stellung eine mit drei Kohlenwasserstoffresten substituierte Silylgruppe, wie Triphenylsilyl-, Dimethylbutyl-silyl- oder vor allem Trimethylsilylgruppe, trägt. Ein solcher $\beta$-(Trihydrocarbylsilyl)-äthoxycarbonylrest, wie ein $\beta$-(Triniederalkylsilyl)-äthoxycarbonyl, z. B. insbesondere das $\beta$-(Trimethylsilyl)-äthoxycarbonyl, bildet mit der zu schützenden $\omega$-Aminogruppe eine entsprechende $\beta$-Trihydrocarbylsilyläthoxycarbonylaminogruppe (z. B. die Trimethylsilyläthoxycarbonylaminogruppe). Dieser Rest ist zwar unter den Bedingungen der sauren Hydrolyse und der Hydrogenolyse beständig, läßt sich aber unter ganz spezifischen, sehr milden Bedingungen durch Einwirkung von Fluoridionen abspalten. In dieser Beziehung verhält er sich analog wie die weiter unten als Carboxyl-Schutzgruppe beschriebene $\beta$-Silyläthylestergruppe. (Diese Ähnlichkeit muß bei der Synthese besonders berücksichtigt werden: bis auf Einzelfälle schließt die Anwendung einer dieser Schutzgruppen die gleichzeitige Anwendung der anderen Schutzgruppe aus.) Weitere Einzelheiten sind weiter unten beim Schutz der Carboxylgruppe durch $\beta$-Silyläthylester angegeben. — Als $X_o$ kommen letztlich auch acidolytisch abspaltbare Gruppen des Aralkyl-Typs, wie Benzhydryl und Triphenylmethyl (Trityl), in Betracht.

Als die Hydroxyl-Schutzgruppe Y können alle zu diesem Zwecke in der Peptid-Chemie gebräuchlichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert-Butyl sowie auch tert-Butoxycarbonyl. Ferner kann man aber auch reduktiv oder basisch abspaltbare Hydroxyl-Schutzgruppen verwenden, z. B. Benzyl- und Benzyloxycarbonylgruppen, die im aromatischen Teil durch Halogen, Nitro und/oder Niederalkoxy substituiert sein können, bzw. Niederalkanoylreste, wie Acetyl, oder Aroylreste, wie Benzoyl. Es ist auch möglich, unter Einhaltung gewisser einschränkender Maßnahmen ohne Schutz der Hydroxylgruppen zu verfahren.

Als die Carboxyl-Schutzgruppe W kann jede übliche zu diesem Zwecke gebräuchliche Gruppe verwendet werden, vgl. das oben zitierte Werk (Houben-Weyl). So werden Carboxylgruppen beispielsweise durch Hydrazidbildung oder durch Veresterung geschützt. Zur Veresterung geeignet sind z. B. niedere, gegebenenfalls substituierte Alkanole wie Methanol, Äthanol, Cyanmethylalkohol, 2,2,2-Trichloräthanol, Benzoylmethylalkohol oder insbesondere tert-Butylalkohol, aber auch ein gegebenenfalls substituierter Benzylalkohol. Eine besonders vorteilhafte Kategorie der substituierten Alkanole sind Äthylalkohole, die in $\beta$-Stellung eine trisubstituierte Silylgruppe, wie Triphenylsilyl-, Dimethyl-butylsilyl- oder vor allem Trimethylsilylgruppe, tragen. Wie z. B. im belgischen Patent Nr. 851 576 beschrieben ist, eignen sich diese Alkohole zum Schutze der Carboxylgruppen deshalb besonders gut, weil die entsprechenden $\beta$-Silyläthylester, z. B. $\beta$-(Trimethylsilyl)-äthylester, zwar die Stabilität üblicher Alkylester besitzen, sich jedoch unter milden Bedingungen durch Einwirkung von Fluoridionen unter Erhaltung aller anderen Schutzgruppen selektiv abspalten lassen.

Vorzugsweise werden die Schutzgruppen $X_o$, Y und W so gewählt, daß sie unter ähnlichen Bedingungen abspaltbar sind; besonders bevorzugt sind dabei die bereits hervorgehobenen acidolytisch abspaltbaren Gruppen. Die Abspaltung aller dieser Schutzgruppen erfolgt dann vorteilhaft in einer einzigen Operation; es können jedoch auch Gruppen verschiedener Art verwendet werden und jede einzeln abgespalten werden. — Wenn z. B. ein Endstoff der Formel I, in welchem $Ac_A$ und $Ac_B$ verschieden sind, durch nachträgliches Acylieren einer freigesetzten Aminogruppe der Reste $Aaa^5$ bzw. $Lys^9$ hergestellt werden soll, so werden beide Schutzgruppen $X_o$ in den Resten $Ac_a$ und $Ac_b$ so gewählt, daß eine davon unter Erhaltung der anderen, und vorzugsweise auch der vorhandenen Schutzgruppen W und insbesondere Y, abspaltbar ist. In diesem Fall wird zunächst die erste (meistens labilere) Schutzgruppe $X_0$ selektiv abgespalten und die freigesetzte Aminogruppe acyliert, nachher dann wird die zweite Aminogruppe freigesetzt und gewünschtenfalls auch acyliert. Die Schutzgruppen W und vor allem Y werden mit Vorteil so gewählt, daß sie erst in der Schlußphase, d. h. nach der letzten Acylierung, abgespalten werden. Solche Kombinationen von Aminoschutzgruppen, die gegeneinander (und auch im bezug auf andere Schutzgruppen vom Typ W und Y) selektiv abspaltbar sind, sind in der Peptid-Chemie allgemein bekannt und gebräuchlich, z. B. reduktiv abspaltbare Gruppen einerseits und acidolytisch abspaltbare Gruppen andererseits.

Die Abspaltung der Schutzgruppen erfolgt in der allgemein bekannten Weise; die saure Hydrolyse (Acidolyse) wird z. B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z. B. durch Hydrierung unter Palladium-Katalyse, entfernt. Die 4-Pyridylmethoxycarbonylgruppe wird vorzugsweise durch Zink-Reduktion abgespalten.

Die gewünschtenfalls durchzuführende nachträgliche Acylierung von vorhandenen freien Aminogruppen in Resten $Aaa^5$ und/oder $Lys^9$ des Cyclopeptids kann vorzugsweise unter vorübergehendem Schutz einer vorhandenen freien Hydroxylgruppe durchgeführt werden. Die Acylierung erfolgt insbesondere dadurch, daß man ein erhaltenes Cyclopeptid der Formel

$$\overline{\underline{|\text{—Aaa(}Ac_A\text{)-Phe-Phe-trp-Lys(}Ac_B\text{)-Thr(}Y_0\text{)-Phe-Gaba—}|}} \qquad \text{(III)}$$

worin $Y_0$ Wasserstoff oder eine Hydroxylschutzgruppe Y der oben näher beschriebenen Bedeutung ist und trp, Aaa, $Ac_A$ und $Ac_B$ die oben definierte Bedeutung mit der Maßgabe haben, daß mindestens einer der Reste $Ac_A$ und $Ac_B$ Wasserstoff ist und im anderen vorhandene Amino- und/oder Hydroxylgruppen in vorübergehend geschützter Form vorliegen, mit einer Carbonsäure $Ac_0OH$, worin $Ac_0$ einen den oben definierten Resten Ac bzw. $Ac^1$ entsprechenden Rest bedeutet, in welchem vorhandene Amino- und Hydroxylgruppen Schutzgruppen $X_0$, $X_a$ bzw. Y tragen können, oder mit einem reaktionsfähigen Derivat einer solchen Säure behandelt und, wenn erwünscht oder notwendig ist, im erhaltenen Produkt durch Abspaltung der Schutzgruppen $X_0$, $X_a$ und Y Aminogruppen bzw. Hydroxylgruppen freisetzt.

Die Bedeutung des Symbols $X_a$, welches eine $\alpha$-Aminoschutzgrupppe bezeichnet, wird weiter unten bei der Synthese der Peptidkette ausführlich beschrieben. Vorzugsweise werden gleichartige Schutzgruppen sowohl im Rest $Y_0$ wie auch $Ac_0$ verwendet und im Anschluß an die Acylierungsreaktion gleichzeitig abgespalten.

Ein reaktionsfähiges Derivat der Säure $Ac_0OH$ ist beispielsweise ein Anhydrid, insbesondere ein symmetrisches Anhydrid der Formel $Ac_0{-}O{-}Ac_0$ oder aber ein gemischtes Anhydrid mit einer anderen organischen Säure, z. B. mit Trifluoressigsäure, oder insbesondere mit einer anorganischen Säure, z. B. ein Säureazid oder Säurehalogenid, vor allem Säurechlorid. Ein reaktionsfähiges Säurederivat ist vorzugsweise ein aktivierter Ester, z. B. ein solcher, in welchem die Säure $Ac_0OH$ mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenzotriazol oder N-Hydroxypiperidin, oder aber mit einem N,N'-disubstituierten Isoharnstoff, wie insbesondere N,N'-Dicyclohexylisoharnstoff oder einer ähnlichen aus der Peptidchemie bekannten Aktivierungskomponente, vgl. Houben-Weyl: Methoden der organischen Chemie; 4. Auflage, Band 15/I und II, E. Wünsch (Herausgeber): Synthese von Peptiden (Georg Thieme Verlag, Stuttgart; 1974), verestert wird.

Die Acylierung erfolgt in an sich bekannter Weise, vorzugsweise in üblichen Lösungsmitteln, beispielsweise Dioxan, Tetrahydrofuran, Acetonitril, Pyridin, Dimethylformamid, Dimethylacetamid, sowie Chloroform und Methylenchlorid, und in zweckmäßigen Gemischen davon. Man kann auch mit einem Zusatz einer organischen Base, z. B. eines quaternären oder vor allem tertiären Amins, wie Triäthylamin, N-Äthylmorpholin oder N-Methylpiperidin, arbeiten, um die zu acylierende Aminogruppe in entprotonisierter Form zu erhalten. Die Reaktionstemperatur beträgt üblicherweise −20 bis +70°C, vorzugsweise etwa 0°C bis Raumtemperatur.

Als Acylierungsmittel sind im allgemeinen Aktivester besonders vorteilhaft, da sie Aminogruppen bevorzugt vor Hydroxylgruppen acylieren und deshalb den Schutz von Hydroxylgruppen praktisch überflüssig machen. Um eine unerwünschte O-Acylierung zu vermeiden, verwendet man üblicherweise nur ein Äquivalent des Acylierungsmittels. Wenn es jedoch aus irgendeinem Grunde vorteilhafter ist, auf die selektive Acylierung zu verzichten, wie es insbesondere bei Umsetzung mit Säurechloriden der Fall sein kann, so nimmt man das Acylierungsmittel im Überschuß und setzt die mitacylierten Hydroxylgruppen nachträglich in gleicher konventioneller Weise wie die geschützten Hydroxylgruppen frei, insbesondere durch basische Hydrolyse, z. B. mit Natrium- oder Kaliumhydroxid in Anwesenheit von Wasser.

Diejenigen erfindungsgemäßen Endstoffe, die basische Gruppen enthalten, werden, je nach Art der Isolierung, als Basen oder als Säureadditionssalze erhalten; diese können nachträglich in an sich

bekannter Weise ineinander umgewandelt werden. Analogerweise können Endstoffe mit sauren Gruppen auch in Form von Salzen vorliegen, wobei beide Formen ineinander in bekannter Weise überführbar sind.

Auch die Bildung der oben erwähnten Komplexe erfolgt nach bekannten Methoden: Komplexe mit schwerlöslichen Metall-, z. B. Aluminium- oder Zinkverbindungen werden vorzugsweise in analoger Weise, wie für ACTH bekannt, z. B. durch Umsetzung mit einem löslichen Salz des betreffenden Metalls, z. B. Zinkchlorid oder Zinksulfat, und Ausfällung mit einem Alkalimetallphosphat und/oder -hydroxid hergestellt. Komplexe mit organischen Verbindungen wie Polyoxygelatine, Carboxymethylcellulose, Polyvinylpyrrolidon, Polyphloretinphosphat, Polyglutaminsäure etc. werden durch Mischen dieser Substanzen mit dem Peptid in wässeriger Lösung erhalten. In gleicher Weise können auch unlösliche Verbindungen mit Alkalimetallpolyphosphaten hergestellt werden.

Die Ausgangsstoffe der oben charakterisierten Formel II sowie die zu ihrer Synthese dienenden, in den Beispielen beschriebenen Zwischenprodukte sind neu und zum Teil auch zur Synthese anderer Somatostatin-Analogen, z. B. solcher mit analogen Aminosäure-Teilsequenzen, mit Vorteil anwendbar. Sie gehören, sowie auch ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CO—NH-Bindungen in beliebiger zeitlicher Reihenfolge miteinander kondensiert, wobei nicht an der Reaktion teilnehmende funktionelle Gruppen intermediär geschützt werden können.

Bei der Herstellung dieser Ausgangsstoffe, wie auch aller benötigten Zwischenprodukte, kommen als Schutzgruppen für die endständigen $\alpha$-Amino- und Carboxylgruppen besonders die bei der Synthese langkettiger Peptide üblichen Schutzgruppen in Betracht, die z. B. durch Solvolyse oder Reduktion leicht und selektiv abgespalten werden können. Sie wurden vorstehend unter der Bezeichnung $X_a$ bzw. W bereits mehrmals erwähnt.

Als $\alpha$-Amino-Schutzgruppe $X_a$ sind beispielsweise zu nennen: gegebenenfalls (z. B. durch Halogen, Nitro, Niederalkyl oder Niederalkoxy) substituierte Di- oder Triarylniederalkylgruppen (wie Diphenylmethyl- oder Triphenylmethylgruppen, z. B. Benzhydryl, Trityl, Di-(p-methoxy)-benzhydryl) oder vor allem von der Kohlensäure sich ableitende hydrogenolytisch abspaltbare Gruppen, wie gegebenenfalls im aromatischen Rest durch Halogenatome, Nitrogruppen, Niederalkyl- oder Niederalkoxygruppen substituierte Benzyloxycarbonylgruppen [z. B. Benzyloxycarbonyl (d. h. Carbobenzoxy), p-Brom- oder p-Chlorbenzyloxycarbonyl, p-Nitrobenzyloxycarbonyl, p-Methoxybenzyloxycarbonyl]; ferner auch 2-(p-Biphenylyl)-2-propyloxycarbonyl und ähnliche im schweizerischen Patent 509 266 beschriebenen Aryloxycarbonylgruppen. Dabei ist zu beachten, daß die $\alpha$-Aminoschutzgruppe $X_a$ selektiv unter Erhaltung der vorhandenen $\omega$-Aminoschutzgruppen $X_o$ der Reste $Aaa^5$ und $Lys^9$ abspaltbar sein muß. Es ist übrigens von Vorteil, wenn bei ihrer Abspaltung auch eine gegebenenfalls vorhandene Carboxyl- und Hydroxylschutzgruppe W bzw. Y unversehrt bleibt.

Die Carboxyl-Schutzgruppen für diesen Zweck sind dieselben, wie sie oben bei der entsprechenden Bedeutung des Symbols W besprochen wurden; vorzugsweise sollen sie auch selektiv, unter Erhaltung übriger Schutzgruppen anderer Art, abspaltbar sein.

Die Schutzgruppe $X_a$ kann in bekannter Weise abgespalten werden. So kann man eine Benzyloxycarbonylgruppe durch Hydrogenolyse, die N-Tritylgruppe mit Mineralsäuren, wie Halogenwasserstoffsäuren (z. B. Fluorwasserstoff oder vorzugsweise Chlorwasserstoff) oder eine organischen Säure (wie Ameisensäure, Essigsäure, Chloressigsäure oder Trifluoressigsäure) in wäßrigem oder absolutem Trifluoräthanol als Lösungsmittel (vgl. deutsche Offenlegungsschrift DE 2 346 147) oder mit wässeriger Essigsäure entfernen; die tert-Butyloxycarbonylgruppe wird z. B. mit Trifluoressigsäure oder Salzsäure und die 2-(p-Biphenylyl)-isopropyloxycarbonylgruppe mit wässeriger Essigsäure oder z. B. mit einem Gemisch von Eisessig, Ameisensäure (82,8%ig) und Wasser (7 : 1 : 2) oder nach dem Verfahren der DE 2 346 147 abgespalten.

Die $\beta$-Silyläthylestergruppen werden vorzugsweise mit Fluoridionen-abgebenden Reagentien, z. B. Fluoriden quaternärer organischer Basen, wie Tetraäthylammoniumfluorid, abgespalten. Sie können aber auch, gleich wie die üblichen Alkylester, durch alkalische Hydrolyse, z. B. mittels Alkalimetallhydroxiden, -carbonaten oder -bicarbonaten, abgespalten werden oder durch Hydrazinolyse, z. B. mittels Hydrazinhydrat, in die entsprechenden Carbazoylgruppen umgewandelt werden. Zur Abspaltung von tert-Butylestern verwendet man vorzugsweise Acidolyse, für Benzylester dann Hydrogenolyse.

Die zur Herstellung der Ausgangsstoffe der Formel II durchzuführende Kondensation der Aminosäure- und/oder Peptideinheiten erfolgt in an sich bekannter Weise, indem man vorzugsweise eine Aminosäure oder ein Peptid mit geschützter $\alpha$-Aminogruppe und freier oder aktivierter terminaler Carboxylgruppe (= aktive Komponente) an eine Aminosäure oder ein Peptid mit freier $\alpha$-Aminogruppe und freier oder, vorzugsweise, geschützter (z. B. veresterter) terminaler Carboxylgruppe (= passive Komponente), anknüpft. Im gebildeten Produkt wird die terminale Aminogruppe freigesetzt und dieses Peptid, enthaltend eine freie $\alpha$-Aminogruppe und eine gegebenenfalls geschützte terminale Carboxylgruppe, wieder mit einer weiteren aktiven Komponente, d. h. einer Aminosäure oder einem Peptid mit aktivierter terminaler Carboxylgruppe und geschützter $\alpha$-Aminogruppe, umsetzt usw. Die Carboxylgruppe kann beispielsweise durch Überführung in ein Säureazid, -anhydrid, -imidazolid, -isoxazolid oder einen aktivierten Ester, wie einen der weiter unten genannten, oder durch Reaktion mit einem Carbodiimid, wie

N,N'-Dicyclohexylcarbodiimid [und gewünschtenfalls unter Zusatz von N-Hydroxysuccinimid, einem unsubstituierten oder einen (z. B. durch Halogen, Methyl oder Methoxy) substituierten 1-Hydroxybenzotriazol oder 4-Hydroxybenzo-1,2,3-triazin-3-oxid], oder insbesondere von N-Hydroxy-5-norbornen-2,3-dicarboximid, oder mit N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode sowie die Merrifield-Methode und die Methode der N-Carboxyanhydride oder N-Thiocarboxyanhydride. Zur Bildung von aktivierten Estern, wie oben erwähnt sind, eignen sich z. B. gegebenenfalls durch elektronenanziehende Substituenten substituierte Phenole und Thiophenole, wie Phenol, Thiophenol, Thiokresol, p-Nitrothiophenol, 2,4,5- und 2,4,6-Trichlorphenol, Pentachlorphenol, o- und p-Nitrophenol, 2,4-Dinitrophenol, p-Cyanophenol, weiter z. B. N-Hydroxysuccinimid, N-Hydroxyphthalimid und N-Hydroxypiperidin.

Bei einer besonders bevorzugten Herstellung der Peptide der Formel II verwendet man als Kupplungsmethode die Carbodiimid-Methode mit N,N'-Dicyclohexylcarbodiimid in Anwesenheit von 1-Hydroxybenzotriazol. Die terminale Carboxylgruppe liegt dabei in Form des $\beta$-(Trimethylsilyl)-äthylesters vor, die $\alpha$-Aminogruppe der aktiven Komponente wird jeweils durch die Benzyloxycarbonylgruppe geschützt, die nach jedem Kupplungsschritt durch Hydrogenolyse abgespalten wird. Zum Schutz der $\omega$-Aminogruppe im Rest $Aaa^5$ und $Lys^9$ sowie sämtlicher Aminogruppen der Reste Ac und $Ac^1$ wird die Acylierung mit der tert-Butoxycarbonylgruppe und für die Hydroxylgruppe der Threoninreste die Verätherung mit der tert-Butylgruppe mit Vorteil verwendet. Diese beiden Schutzgruppen können, wenn erwünscht, zuletzt in einem Schritt durch saure Hydrolyse, z. B. mittels Trifluoressigsäure, Salzsäure oder Fluorwasserstoff, abgespalten werden. Wenn die $\omega$-Aminogruppe des Restes $Aaa^5$ bzw. $Lys^9$ vom Anfang an durch Ac bzw. $Ac^1$ acyliert ist, benötigt sie keinen Schutz.

Je nach Arbeitsweise erhält man die Verbindungen der Formel II, je nach ihrem Charakter, in Form von Basen oder Säureadditionssalzen, oder aber von Säuren oder ihren Salzen. Aus den Säureadditionssalzen können in an sich bekannter Weise die Basen gewonnen werden. Von letzteren wiederum lassen sich durch Umsetzen mit Säuren, z. B. mit solchen, die die oben genannten Salze bilden, therapeutisch anwendbare Säureadditionssalze gewinnen. In ähnlicher Beziehung stehen auch Säuren und ihre Salze zueinander. Innere Salze werden bei Verbindungen mit entsprechender Struktur durch Einstellen von pH auf einen geeigneten Neutralpunkt gewonnen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäß gegebenenfalls auch die entsprechenden Salze bzw. freien Verbindungen zu verstehen.

Die Erfindung betrifft auch diejenigen Ausführungsformen der Verfahren, bei denen man von einer auf beliebiger Verfahrensstufe als Zwischenprodukt erhältlichen Verbindung ausgeht und die fehlenden Verfahrensschritte ausführt oder wobei ein Ausgangsstoff unter den Reaktionsbedingungen gebildet oder in Form eines Derivats davon, gegebenenfalls eines Salzes, verwendet wird.

Unter derartigen Ausführungsformen des erfindungsgemäßen Verfahrens ist insbesondere diejenige hervorzuheben, bei welcher man die Endstoffe der eingangs definierten Formel I so herstellt, daß man in entsprechenden Zwischenprodukten, die z. B. durch Cyclisierung zugänglich sind und in welchen mindestens eine der vorhandenen Amino-, Hydroxyl- und/oder Carboxylgruppen in geschützter Form vorliegen, diese Gruppen freisetzt. Diese Ausführungsform, die ganz allgemein eine Verbindung der Formel

$$\overline{\underline{\quad Aaa(Ac_A)\text{-}Phe\text{-}Phe\text{-}trp\text{-}Lys(Ac_B)\text{-}Thr\text{-}Phe\text{-}Gaba \quad}} \qquad (I)$$

worin

- Aaa der Rest einer geradkettigen $\alpha,\omega$-Diaminoalkansäure mit 4—7 C-Atomen,
- trp der Rest L-Trp oder insbesondere D-Trp, oder ein davon abgeleiteter Rest, der im Indolkern ein Halogen trägt,
- $Ac_A$ ein an der $\omega$-Aminogruppe befindlicher Acylrest Ac einer gegebenenfalls substituierten Carbonsäure, die Amidinogruppe oder Wasserstoff, und
- $Ac_B$ ein an der $\varepsilon$-Aminogruppe befindlicher Acylrest $Ac^1$ einer Aminosäure oder eines Oligopeptids, oder vorzugsweise Wasserstoff

ist, oder ein Salz oder ein Komplex davon ergibt, ist dadurch gekennzeichnet, daß man in einer strukturell entsprechenden Verbindung, in welcher mindestens eine von vorhandenen Amino-, Hydroxyl- und/oder Carboxylgruppen eine Schutzgruppe $X_0$, $X_a$, Y, bzw. W trägt, vorhandene Schutzgruppen abspaltet und, wenn erwünscht, eine erhaltene basische Verbindung in ein Säureadditionssalz oder ein erhaltenes Säureadditionssalz in die entsprechende Base umwandelt und/oder eine erhaltene Verbindung in ihr Komplex umwandelt.

Bevorzugte Schutzgruppen $X_0$, $X_a$, Y und W wurden bereits oben besprochen; vornehmlich werden solche Kombinationen davon gewählt, die unter gleichen Bedingungen abspaltbar sind, vorzugsweise z. B. oben beschriebene acidolytisch abspaltbare Reste (wie insbesondere die vom tert-Butyl-Typ).

Beim Verfahren der vorliegenden Erfindung werden vorzugsweise solche Ausgangsstoffe verwendet,

welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze oder Komplexe davon enthalten. Diese Präparate können insbeondere in den oben angegebenen Indikationen Verwendung finden, wenn sie parenteral (wie intravenös, intramuskulär oder subcutan) oder auch intranasal oder oral verabreicht werden. Die Dosierung hängt von der spezifischen zu behandelnden Erkrankung, ihrer Schwere und von der Dauer der Therapie ab. Anzahl und Menge der Einzeldosen sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,0015 bis etwa 0,15 mg/kg Körpergewicht, und die Verabreichung erfolgt durch intravenöse Infusion oder subkutane Injektion. Dementsprechend enthalten pharmazeutische Präparate zur parenteralen Verabreichung in Einzeldosis-Form in Abhängigkeit von der Applikationsart pro Dosis etwa 0,08 bis etwa 15 mg einer der erfindungsgemäßen Verbindungen. Neben dem Wirkstoff enthalten sie üblicherweise noch einen Puffer, z. B. einen Phosphatpuffer, der das pH zwischen etwa 3,5 und 7 halten soll, ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z. B. 0,2—0,3% 4-Hydroxybenzoesäure-methylester oder -äthylester, enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z. B. gemäß den oben erwähnten Maßnahmen zubereitet werden, gebildet werden. Als Zusatz eignet sich z. B. 0,1— 1,0 Gewichts-% eines Zink(II)-Salzes (z. B. Sulfats) in Verbindung mit 0,5—5,0 Gewichts-% Protamin (z. B. als Sulfat), bezogen auf das Gesamtvolumen der Injektionslösung; diese Zubereitung liegt als Lösung von pH 3,5 bis etwa 6,5 oder als Suspension von etwa pH 7,5 bis 8,0 vor.

Ein Präparat für die intranasale Verabreichung kann als eine wäßrige Lösung oder Gelee, eine ölige Lösung oder Suspension oder aber eine fetthaltige Salbe vorliegen. Ein Präparat in Form einer wäßrigen Lösung erhält man z. B. dadurch, daß man den Wirkstoff der Formel I, oder ein therapeutisch anwendbares Salz oder Komplex davon, in einer wäßrigen Pufferlösung von pH bis 7,2 löst und einen Isotonieerzeugenden Stoff zusetzt. Der wäßrigen Lösung wird zweckmäßig ein polymeres Haftmittel, z. B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z. B. durch Suspendieren eines Peptids der Formel I oder eines therapeutisch anwendbaren Säureadditionssalzes davon in einem Öl, gegebenenfalls unter Zusatz von Quellmitteln, wie Aluminiumstearat, und/oder grenzflächenaktiven Mitteln (Tensiden), deren HLB-Wert (»hydrophilic-lipophilic-balance«) unter 10 liegt, wie Fettsäuremonoester mehrwertiger Alkohole, z. B. Glycerinmonostearat, Sorbitanmonolaurat, Sorbitanmonostearat oder Sorbitanmonooleat. — Eine fetthaltige Salbe erhält man z. B. durch Suspendieren des erfindungsgemäßen Wirkstoffes in einer streichbaren Fettgrundlage, gegebenenfalls unter Zusatz eines Tensids vom HLB-Wert unter 10. Eine Emulsionssalbe erhält man durch Verreiben einer wäßrigen Lösung des peptidischen Wirkstoffes in einer weichen, streichbaren Fettunterlage unter Zusatz eines Tensids, dessen HLB-Wert unter 10 liegt. Alle diese intranasalen Applikationsformen können auch Konservierungsmittel enthalten. — Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten außer dem Wirkstoff üblicherweise Hilfsmittel: Insufflationskapseln enthalten z. B. feste Trägerstoffe, wie Lactose; Aerosol- bzw. Sprayzubereitungen enthalten z. B. ein flüssiges Treibgas mit einem Siedepunkt unter der Raumtemperatur sowie, wenn erwünscht, weitere Trägerstoffe, wie flüssige oder feste nicht-ionische oder anionische Tenside und/oder feste Verdünnungsmittel. Präparate, in welchen der pharmakologische Wirkstoff in Lösung vorliegt, enthalten außer diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbindungen der Formel I und therapeutisch anwendbarer Salze und Komplexe davon als pharmakologisch aktive Verbindungen, insbesondere in den eingangs angegebenen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen werden einige besonders vorteilhafte Durchführungsvarianten der Erfindung noch näher illustriert, ohne jedoch diese dadurch einzuschränken. Temperaturen werden in Celsiusgraden angegeben; als Abkürzungen, z. B. zur Bezeichnung von Aminosäuren, Peptiden, Schutzgruppen etc., werden die üblichen, z. B. die in »Synthese von Peptiden« (Herausgeber: E. Wünsch), Bd. XV der »Methoder der org. Chemie« (Houben-Weyl) (1974; G. Thieme, Stuttgart), zusammengestellten Kurzbezeichnungen verwendet. Insbesondere werden folgende Abkürzungen verwendet.

| | |
|---|---|
| Boc | — tert-Butoxycarbonyl |
| But | — tert-Butyl (als ätherbildende Gruppe) |
| OBzl | — Benzyloxy (als esterbildende Gruppe) |
| OPc | — Pentachlorphenoxy (als esterbildende Gruppe) |
| OTmse | — 2-(Trimethylsilyl)-äthoxy (als esterbildende Gruppe) |
| Z | — Benzyloxycarbonyl (Carbobenzoxy) |

sowie

| | |
|---|---|
| DC | — Dünnschichtchromatographie |
| DCCI | — Dicyclohexylcarbodiimid |
| DMF | — Dimethylformamid |

In DC verwendet man, wenn nichts anderes angegeben ist, Kieselgel als Adsorbens und die folgenden Systeme als Laufmittel (in Volum-Proportionen).

| | | |
|---|---|---|
| System: | 101: | n-Butanol-Pyridin-Essigsäure-Wasser (38:24:8:30) |
| | 111 B: | n-Butanol-Pyridin-25%iges wäßriges Ammoniak-Wasser (40:24:6:30) |
| | 112 A: | n-Butanol-Pyridin-Ameisensäure-Wasser (42:24:4:20) |
| | 157: | Chloroform-Methanol-Wasser-Essigsäure (70:42:10:0,5) |
| | 157 A: | Chloroform-Methanol-Wasser-Essigsäure (90:10:1:0,5) |
| | 157 B: | Chloroform-Methanol-Wasser-Essigsäure (85:13:1,5:0,5) |
| | 157 G: | Chloroform-Methanol-Wasser-Essigsäure (88:10,5:1,0:0,5) |

## Beispiel 1

cyclo-[Lys-Phe-Phe-(D-Trp)-Lys-Thr-Phe-Gaba]

280 mg geschütztes Octapeptid der Formel

cyclo-[Lys(Boc)-Phe-Phe-(D-Trp)-Lys(Boc)-Thr(But)-Phe-Gaba]

wird bei 5° unter $N_2$ in 1,5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 90 Minuten bei Raumtemperatur unter $N_2$ mit 15 ml Äther ausgefällt. Das resultierende rohe Trifluoracetat des Endprodukts wird in Vakuum getrocknet, in 5 ml 1 N-Essigsäure gelöst und durch 15 ml Anionenaustauscher, z. B. AG® 1-X8 (ein Produkt von Bio-Rad Laboratories, Richmond, Calif., USA), in Acetatform filtriert. Das Eluat wird im Vakuum eingedampft und der Rückstand der Gegenstromverteilung im System tert-Amylalkohol-Essigsäure-Wasser-Toluol (4:2:5:0,8) über 680 Stufen unterzogen. Die in den Elementen 180 bis 220 enthaltenen Phasen (K = 0,4) werden gesammelt, im Vakuum eingedampft und aus tert-Butanol-Wasser (1:1) lyophilisiert.

Die erhaltene Titelverbindung ist in zwei Systemen dünnschicht-chromatographisch einheitlich.

DC: System 157: $R_f$ 0,4; 157 G: $R_f$ 0,6.

Der peptidische Ausgangsstoff ist folgendermaßen erhältlich:

### Stufe 1.1

Z-Lys(Boc)-Phe-Phe-OH

4,19 g Z-Lys(Boc)-OPc und 2,47 g HCl · H-Phe-Phe-OH werden in 50 ml Dimethylformamid aufgenommen, 0,84 ml N-Äthylmorpholin zugegeben und 24 Stunden bei 20° stehengelassen. Das Reaktionsgemisch wird eingedampft und der Rückstand mit Wasser verrieben und über Phosphorpentoxid getrocknet. Umkristallisation aus Äther gibt Kristalle vom Smp. 154 bis 156°.

DC: System 157 A: $R_f$ 0,25.

Stufe 1.2

Z-Lys(Boc)-Phe-Phe-(D-Trp)-Lys(Boc)-Thr(But)-Phe-OTmse

Eine Lösung von 2,1 g Z-Lys(Boc)-Phe-Phe-OH (Stufe 1.1) und 2,51 g H-(D-Trp)-Lys(Boc)-Thr(But)-Phe-OTmse in 35 ml Dimethylformamid wird mit 460 mg 1-Hydroxy-benzotriazol und 680 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 5 ml Methanol verrieben und abgesaugt. Das ungelöste Material wird zur Reinigung nochmals mit 5 ml Methanol bei 50° verrieben, abgesaugt, mit Methanol gewaschen und im Vakuum getrocknet. Das Produkt ist gemäß DC einheitlich.
DC: System (Chloroform-Methanol 9:1): $R_f$ 0,73.

Stufe 1.3

Z-Lys(Boc)-Phe-Phe-(D-Trp)-Lys(Boc)-Thr(But)-Phe-OH

2,93 g Z-Lys(Boc)-Phe-Phe-(D-Trp)-Lys(Boc)-Thr(But)-Phe-OTmse (Stufe 1.2) wird in 75 ml einer frisch hergestellten, wasserfreien 0,15 M-Tetraäthylammonium-fluorid-Lösung in Dimethylformamid gelöst und 30 Minuten bei 25° gehalten. Nach dem Abkühlen auf 5° wird das Reaktionsgemisch unter gutem Umrühren mit 2,0 ml 1 N wässeriger Salzsäure versetzt und das Produkt durch Zugabe von 700 ml Wasser gefällt. Das abfiltrierte Material wird mit Wasser gewaschen, im Vakuum über Phosphorpentoxid getrocknet und direkt weiter verwendet.
DC: System 157 A: $R_f$ 0,30.

Stufe 1.4

Z-Lys(Boc)-Phe-Phe-(D-Trp)-Lys(Boc)-Thr(But)-Phe-Gaba-OBzl

Ein Gemisch von 2,17 g Z-Lys(Boc)-Phe-Phe-(D-Trp)-Lys(Boc)-Thr(But)-Phe-OH (Stufe 1.3) und 740 mg Gaba-benzylester-p-toluolsulfonat in 16 ml Dimethylformamid wird mit 286 mg 1-Hydroxybenzotriazol, 385 mg DCCI und 0,255 ml N-Äthylmorpholin versetzt und 20 Stunden bei Raumtemperatur belassen. Zur Aufarbeitung wird das Gemisch mit 10 ml eiskaltem Methanol versetzt und abfiltriert. Der gewonnene Feststoff wird zur weiteren Reinigung mit 5 ml warmem Methanol während 10 Minuten verrührt, die Suspension auf 0° abgekühlt, das reine Produkt abfiltriert und im Vakuum getrocknet.
DC: [Chloroform-Methanol (95:5)] $R_f$ 0,55.

Stufe 1.5

H-Lys(Boc)-Phe-Phe-(D-Trp)-Lys(Boc)-Thr(But)-Phe-Gaba-OH

Eine Lösung von 2,04 g Z-Lys(Boc)-Phe-Phe-(D-Trp)-Lys(Boc)-Thr(But)-Phe-Gaba-OBzl (Stufe 1.4) in 100 ml Dimethylformamid wird nach Zugabe von 500 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 25 ml peroxidfreiem Äther ausgefällt, abfiltriert und im Vakuum getrocknet. Das Rohprodukt wird ohne weitere Reinigung der nächsten Stufe 1.6 (Cyclisierung) unterzogen.

Stufe 1.6

┌Lys(Boc)-Phe-Phe-(D-Trp)-Lys(Boc)-Thr(But)-Phe-Gaba┐
└──────────────────────────────────┘

Eine Lösung von 630 mg rohem H-Lys(Boc)-Phe-Phe-(D-Trp)-Lys(Boc)-Thr(But)-Phe-Gaba-OH (Stufe 1.5), 650 mg 1-Hydroxybenzotriazol und 875 mg DCCI in 240 ml Dimethylformamid wird während 20 Stunden bei 50° gehalten. Zur Aufarbeitung wird das Lösungsmittel im Hochvakuum bei ca. 30° abgedampft und der Rückstand mit 10 ml Äthylacetat verrieben. Der ausgefallene Dicyclohexylharnstoff wird durch Abfiltrieren beseitigt, das Filtrat mit Äthylacetat auf 50 ml verdünnt, dreimal mit je 20 ml 1 N wässeriger Oxalsäure und dann mit Wasser bis zur Neutralität gewaschen, über Natriumsulfat getrocknet und im Vakuum eingedampft. Das Rohprodukt wird an einer Säule von 50 g Kieselgel (in

Äthylacetat-Petroläther 1 : 1 zubereitet) chromatographiert. Nach einem Vorlauf von 750 ml Äthylacetat-Petroläther 1 : 1 eluieren 500 ml Äthylacetat 410 mg des dünnschichtchromatographisch einheitlichen Produktes.

DC: System 157 A: $R_f$ 0,45.

### Beispiel 2

┌─Orn-Phe-Phe-(D-Trp)-Lys-Thr-Phe-Gaba─┐

1,3 g geschütztes Octapeptid der Formel

┌─Orn(Boc)-Phe-Phe-(D-Trp)-Lys(Boc)-Thr(But)-Phe-Gaba─┐

wird bei 5° unter $N_2$ in 30 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 90 Minuten bei Raumtemperatur unter $N_2$ mit 100 ml Äther ausgefällt. Das resultierende rohe Trifluoracetat des Endprodukts wird im Vakuum getrocknet, in 20 ml 1 N-Essigsäure gelöst und durch 40 ml Anionenaustauscher, z. B. AG® 1-X8 (ein Produkt von Bio-Rad Laboratories, Richmond, Calif., USA), in Acetatform filtriert. Das Eluat wird im Vakuum eingedampft und der Rückstand der Gegenstromverteilung im System tert-Amylalkohol-Essigsäure-Toluol (4 : 2 : 5 : 0,8) über 785 Stufen unterzogen. Die in den Elementen 360 bis 400 enthaltenen Phasen (K = 0,8) werden gesammelt, im Vakuum eingedampft und aus tert-Butanol-Wasser (1 : 1) lyophilisiert.

Die erhaltene Titelverbindung ist in zwei Systemen dünnschichtchromatographisch einheitlich.

DC: System 157: $R_f$ 0,35; 157 G: $R_f$ 0,30.

Der peptidische Ausgangsstoff ist folgendermaßen erhältlich:

### Stufe 2.1

Z-Orn(Boc)-Phe-Phe-OTmse

1,47 g Z-Orn(Boc)-OH, 1,8 g HCl · H-Phe-Phe-OTmse, 900 mg DCCI und 645 mg 1-Hydroxybenzotriazol werden in 30 ml Dimethylformamid aufgenommen, 0,51 ml N-Äthylmorpholin zugegeben und 24 Stunden stehengelassen. Es wird abfiltriert, das Filtrat eingedampft und der Rückstand aus Äthylacetat umkristallisiert. Smp. 147 bis 148°.

DC: System (Toluol-Aceton 7 : 3): $R_f$ 0,40.

### Stufe 2.2

Z-Orn(Boc)-Phe-Phe-OH

1,52 g Z-Orn(Boc)-Phe-Phe-OTmse (Stufe 2.1) und 1,85 g Tetraäthylammonium-fluorid werden in 28 ml Dimethylformamid gelöst und 45 Minuten stehengelassen. Man gibt dann 2 ml 1 N-Salzsäure und 100 ml Wasser zu, filtriert ab und trocknet über Phosphorpentoxid.

DC: System 157 B: $R_f$ 0,45.

### Stufe 2.3

Z-Orn(Boc)-Phe-Phe-(D-Trp)-Lys(Boc)-Thr(But)-Phe-OTmse

Eine Lösung von 1,2 g Z-Orn(Boc)-Phe-Phe-OH (Stufe 2.2) und 1,52 g H-(D-Trp)-Lys(Boc)-Thr(But)-Phe-OTmse in 25 ml Dimethylformamid wird mit 278 mg 1-Hydroxy-benzotriazol und 412 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 10 ml Methanol verrieben und abgesaugt. Das ungelöste Material wird zur Reinigung nochmals mit 10 ml Methanol bei 50° verrieben, abgesaugt, mit Methanol gewaschen und im Vakuum getrocknet. Das Produkt ist gemäß DC einheitlich.

DC: [Chloroform-Methanol (95 : 5)] $R_f$ 0,50.

## Stufe 2.4

Z-Orn(Boc)-Phe-Phe-(D-Trp)-Lys(Boc)-Thr(But)-Phe-OH

2,84 g Z-Orn(Boc)-Phe-Phe-(D-Trp)-Lys(Boc)-Thr(But)-Phe-OTmse (Stufe 2.3) wird in 70 ml einer frisch hergestellten, wasserfreien 0,15 M-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 1,8 ml 1 N wässeriger Salzsäure versetzt und das Produkt durch Zugabe von 500 ml Wasser gefällt. Das abfiltrierte Material wird mit Wasser gewaschen, im Vakuum über Phosphorpentoxid getrocknet und direkt weiter verwendet.

DC: System 157 B: $R_f$ 0,50.

## Stufe 2.5

Z-Orn(Boc)-Phe-Phe-(D-Trp)-Lys(Boc)-Thr(But)-Phe-Gaba-OBzl

Ein Gemisch von 2,3 g Z-Orn(Boc)-Phe-Phe-(D-Trp)-Lys(Boc)-Thr(But)-Phe-OH (Stufe 2.4) und 796 mg Gaba-benzylester-p-toluolsulfonat in 16 ml Dimethylformamid wird mit 307 mg 1-Hydroxybenzotriazol, 413 mg DCCI und 0,273 ml N-Äthylmorpholin versetzt und 20 Stunden bei Raumtemperatur belassen. Zur Aufarbeitung wird das Gemisch mit 30 ml eiskaltem Methanol versetzt und abfiltriert. Der gewonnene Feststoff wird zu weiteren Reinigung mit 15 ml warmem Methanol während 10 Minuten verrührt, die Suspension auf 0° abgekühlt, das reine Produkt abfiltriert und im Vakuum getrocknet.

DC: System 157 B: $R_f$ 0,80.

## Stufe 2.6

H-Orn(Boc)-Phe-Phe-(D-Trp)-Lys(Boc)-Thr(But)-Phe-Gaba-OH

Eine Lösung von 2,6 g Z-Orn(Boc)-Phe-Phe-(D-Trp)-Lys(Boc)-Thr(But)-Phe-Gaba-OBzl (Stufe 2.5) in 125 ml Dimethylformamid wird nach Zugabe von 500 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 100 ml peroxidfreiem Äther ausgefällt, abfiltriert und im Vakuum getrocknet. Das Rohprodukt wird ohne weitere Reinigung der nächsten Stufe unterzogen.

DC: System 157 b: $R_f$ 0,40.

## Stufe 2.7

Orn(Boc)-Phe-Phe-(D-Trp)-Lys(Boc)-Thr(But)-Phe-Gaba

Eine Lösung von 2,08 g rohem H-Orn(Boc)-Phe-Phe-(D-Trp)-Lys(Boc)-Thr(But)-Phe-Gaba-OH (Stufe 2.6), 2,41 g 1-Hydroxybenzotriazol und 3,24 g DCCI in 1570 ml Dimethylformamid wird während 20 Stunden bei 50° gehalten. Zur Aufarbeitung wird das Lösungsmittel im Hochvakuum bei ca. 30° abgedampft und der Rückstand mit 30 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 20 ml 1 N wässeriger Oxalsäure und dann mit Wasser bis zur Neutralität gewaschen, über Natriumsulfat getrocknet und im Vakuum eingedampft. Das Rohprodukt wird zur Reinigung an einer Säule von 75 g Kieselgel (in Chloroform zubereitet) chromatographiert. Nach einem Vorlauf von 200 ml Chloroform eluieren 250 ml Chloroform-Methanol (95 : 5) 1,36 g dünnschichtchromatographisch einheitliches Produkt.

DC: System 157 B: $R_f$ 0,70.

## Beispiel 3

H-Lys-Lys-Lys

Lys-Phe-Phe-(D-Trp)-Lys-Thr-Phe-Gaba

467 mg geschütztes Octapeptid der Formel

Boc-Lys(Boc)-Lys(Boc)-Lys(Boc)—┐

┌—Lys-Phe-Phe-(D-Trp)-Lys(Boc)-Thr(But)-Phe-Gaba—┐
└————————————————————————————————┘

wird bei 5° unter $N_2$ in 5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 90 Minuten bei Raumtemperatur unter $N_2$ mit 40 ml Äther ausgefällt. Das resultierende rohe Trifluoracetat des Endprodukts wird im Vakuum getrocknet, in 5 ml 1 N-Essigsäure gelöst und durch 15 ml Anionenaustauscher, z. B. AG® 1-X8 (ein Produkt von Bio-Rad Laboratories, Richmond. Calif., USA), in Acetatform filtriert. Das Eluat wird im Vakuum eingedampft und der Rückstand der Gegenstromverteilung im System tert-Amylalkohol-Essigsäure-Wasser-Toluol (4 : 2 : 5 : 0,8) über 1280 Stufen unterzogen. Die in den Elementen 400 bis 430 enthaltenen Phasen (K = 0,48) werden gesammelt, im Vakuum eingedampft und aus tert-Butanol-Wasser (1 : 1) lyophilisiert.

Die erhaltene Titelverbindung ist in zwei Systemen dünnschichtchromatographisch einheitlich.

DC: 111 B: $R_f$ 0,1; 112 A: $R_f$ 0,2.

Der peptidische Ausgangsstoff ist folgendermaßen erhältlich:

## Stufe 3.1

### Z-Lys(Boc)-Phe-Phe-OTmse

3,8 g Z-Lys(Boc)-OH, 4,49 g HCl · H-Phe-Phe-OTmse, 1,53 g 1-Hydroxybenzotriazol und 2,26 g DCCI werden in 40 ml Dimethylformamid gelöst, 1,26 ml N-Äthylmorpholin zugegeben und 24 Stunden bei 20° stehengelassen. Das Reaktionsgemisch wird filtriert, das Filtrat eingeengt und das Produkt mit Wasser gefällt. Nach dem Trocknen des Niederschlages über Phosphorpentoxid wird aus Äthylacetat-Petroläther ausgefällt.

DC: System Äthylacetat: $R_f$ 0,65.

## Stufe 3.2

### Z-Lys(HCl)-Phe-Phe-OTmse

775 mg Z-Lys(Boc)-Phe-Phe-OTmse (Stufe 3.1) werden in 25 ml Chlorwasserstoff-Äthylacetat (2,4 N) gelöst und die Lösung nach einer Stunde bei 20° eingedampft. Der Rückstand wird mit Äther verrieben und über Kaliumhydroxid getrocknet.

DC: System 157: $R_f$ 0,60.

## Stufe 3.3

Boc-Lys(Boc)-Lys(Boc)-Lys(Boc)—┐
Z-Lys-Phe-Phe-OTmse

514 mg Boc-Lys(Boc)-Lys(Boc)-Lys(Boc)-OH (siehe die nachfolgende Sequenz der Stufen 3.3 A—D), 665 mg Z-Lys(HCl)-Phe-Phe-OTmse (Stufe 3.2), 157 mg 1-Hydroxy-benzotriazol und 212 mg DCCI werden in 5 ml Dimethylformamid gelöst, 0,118 ml N-Äthylmorpholin zugegeben und 24 Stunden bei 20° stehengelassen. Es wird abfiltriert, das Filtrat eingedampft, der Rückstand in 300 ml Äthylacetat dreimal mit je 50 ml Wasser gewaschen, die organische Phase über Natriumsulfat getrocknet. Man engt die Lösung ein und fällt das Produkt mit 100 ml Petroläther aus.

DC: System (Chloroform-Methanol 93 : 7): $R_f$ 0,40.

## Stufe 3.3 A

### Z-Lys(Boc)-Lys(Boc)-OTmse

Zu 1,89 g Z-Lys(Boc)-OPc und 1,15 g HCl · H-Lys(Boc)-OTmse in 30 ml Dimethylformamid werden 0,378 ml N-Äthylmorpholin gegeben. Nach 24 Stunden bei 20° engt man ein und fällt mit 100 ml Wasser aus. Das über Phosphorpentoxid getrocknete Produkt wird durch Umfällen aus Äthylacetat-Petroläther gereinigt.

DC: System (Toluol-Aceton 7:3): $R_f$ 0,55.

Stufe 3.3 B

HCl · H-Lys(Boc)-Lys(Boc)-OTmse

Eine Lösung von 1,33 g Z-Lys(Boc)-Lys(Boc)-OTmse (Stufe 3.3 A) in 50 ml Methanol und 1,883 ml 1 N wässerige Salzsäure wird nach Zugabe von 130 mg Palladium-Kohle (10%) während einer Stunde bei Raumtemperatur und Normaldruck hydriert. Der Katalysator wird abfiltriert, die Lösung eingedampft und das erhaltene Produkt direkt weiter verwendet.
DC: System 157 A: $R_f$ 0,15.

Stufe 3.3 C

Boc-Lys(Boc)-Lys(Boc)-OTmse

Zu 803 mg Boc-Lys(Boc)-OH in 10 ml Dimethylformamid gibt man bei −15° 0,285 ml N-Äthylmorpholin und 0,3 ml Isobutylchlorocarbonat. Nach 15 Minuten bei −15° gibt man die Lösung von 1,51 g HCl · H-Lys(Boc)-Lys(Boc)-OTmse (Stufe 3.3 B) und 0,237 ml N-Äthylmorpholin in 15 ml Dimethylformamid zu. Nach 1 Stunde bei −10° und 24 Stunden bei 20° dampft man ein, nimmt den Rückstand in 100 ml Äthylacetat auf und wäscht dreimal mit je 20 ml Wasser. Die organische Phase wird über Natriumsulfat getrocknet, abfiltriert, das Filtrat eingeengt und das Produkt durch Zugabe von Petroläther ausgefällt.
DC: System (Toluol-Aceton 7:3): $R_f$ 0,30.

Stufe 3.3 D

Boc-Lys(Boc)-Lys(Boc)-Lys(Boc)-OH

1,5 g Boc-Lys(Boc)-Lys(Boc)-Lys(Boc)-OTmse (Stufe 3.3 C) und 1,53 g Tetraäthylammoniumfluorid werden in 20 ml Dimethylformamid gelöst. Nach 45 Minuten bei 20° gibt man 1,66 ml 1 N wässerige Salzsäure und 100 ml Wasser zu, filtriert ab und trocknet über Phosphorpentoxid.
DC: System 157 B: $R_f$ 0,40.

Stufe 3.4

Boc-Lys(Boc)-Lys(Boc)-Lys(Boc)—┐
Z-Lys-Phe-Phe-OH

Boc-Lys(Boc)-Lys(Boc)-Lys(Boc)—┐
834 mg Z-Lys-Phe-Phe-OTmse

(Stufe 3.3) und 530 mg Tetraäthylammoniumfluorid werden in 7 ml Dimethylformamid 45 Minuten bei 20° stehengelassen. Man gibt dann 0,571 ml 1 N wässerige Salzsäure und 75 ml Wasser zu, filtriert ab und trocknet über Phosphorpentoxid.
DC: System 157 B: $R_f$ 0,40.

Stufe 3.5

Boc-Lys(Boc)-Lys(Boc)-Lys(Boc)—┐
Z-Lys-Phe-Phe-(D-Trp)-Lys(Boc)-Thr(But)-Phe-OTmse

Eine Lösung von 740 mg

Boc-Lys(Boc)-Lys(Boc)-Lys(Boc)

Z-Lys-Phe-Phe-OH

(Stufe 3.4) und 455 mg H-(D-Trp)-Lys(Boc)-Thr(But)-Phe-OTmse in 10 ml Dimethylformamid wird mit 84 mg 1-Hydroxy-benzotriazol und 123 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 5 ml Methanol verrieben und abgesaugt. Das ungelöste Material wird zur Reinigung nochmals mit 5 ml Methanol bei 50° verrieben, abgesaugt, mit Methanol gewaschen und im Vakuum getrocknet. Das Produkt ist gemäß DC einheitlich.
DC: [Chloroform-Methanol (9 : 1)] $R_f$ 0,60.

Stufe 3.6

Boc-Lys(Boc)-Lys(Boc)-Lys(Boc)

Z-Lys-Phe-Phe-(D-Trp)-Lys(Boc)-Thr(But)-Phe-OH

Boc-Lys(Boc)-Lys(Boc)-Lys(Boc)

1,04 g Z-Lys-Phe-Phe-(D-Trp)-Lys(Boc)-Thr(But)-Phe-OTmse

(Stufe 3.5) wird in 25 ml einer frisch hergestellten, wasserfreien 0,15 M-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 0,48 ml 1 N wässeriger Salzsäure versetzt und das Produkt durch Zugabe von 70 ml Wasser gefällt. Das abfiltrierte Material wird mit 5 ml Wasser gewaschen, im Vakuum über Phosphorpentoxid getrocknet und direkt weiter verwendet.
DC: System 157 B: $R_f$ 0,45.

Stufe 3.7

Boc-Lys(Boc)-Lys(Boc)-Lys(Boc)

H-Lys-Phe-Phe-(D-Trp)-Lys(Boc)-Thr(But)-Phe-Gaba-OBzl

Ein Gemisch von 957 mg

Boc-Lys(Boc)-Lys(Boc)-Lys(Boc)

Z-Lys-Phe-Phe-(D-Trp)-Lys(Boc)-Thr(But)-Phe-OH

(Stufe 3.6) und 219 mg Gaba-benzylester-p-toluolsulfonat in 7 ml Dimethylformamid wird mit 85 mg 1-Hydroxybenzotriazol, 114 mg DCCI und 0,075 ml N-Äthylmorpholin versetzt und 20 Stunden bei Raumtemperatur belassen. Zur Aufarbeitung wird das Gemisch mit 10 ml eiskaltem Methanol versetzt und abfiltriert. Der gewonnene Feststoff wird zur weiteren Reinigung mit 5 ml warmem Methanol während 10 Minuten verrührt, die Suspension auf 0° abgekühlt, das reine Produkt abfiltriert und im Vakuum getrocknet.
DC: [Chloroform-Methanol (9 : 1)] $R_f$ 0,60.

Stufe 3.8

Boc-Lys(Boc)-Lys(Boc)-Lys(Boc)

Z-Lys-Phe-Phe-(D-Trp)-Lys(Boc)-Thr(But)-Phe-Gaba-OH

Eine Lösung von 1,0 g

Boc-Lys(Boc)-Lys(Boc)-Lys(Boc)

Z-Lys-Phe-Phe-(D-Trp)-Lys(Boc)-Thr(But)-Phe-Gaba-OBzl

(Stufe 3.7) in 50 ml Dimethylformamid wird nach Zugabe von 150 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 50 ml peroxidfreiem Äther ausgefällt, abfiltriert und im Vakuum getrocknet. Das Rohprodukt wird ohne weitere Reinigung der nächsten Stufe unterzogen.

DC: System 157 A: $R_f$ 0,30.

Stufe 3.9

Boc-Lys(Boc)-Lys(Boc)-Lys(Boc)

Lys-Phe-Phe-(D-Trp)-Lys(Boc)-Thr(But)-Phe-Gaba

Eine Lösung von 913 mg rohem

Boc-Lys(Boc)-Lys(Boc)-Lys(Boc)

H-Lys-Phe-Phe-(D-Trp)-Lys(Boc)-Thr(But)-Phe-Gaba-OH

(Stufe 3.8), 678 mg 1-Hydroxybenzotriazol und 1,0 g DCCI in 240 ml Dimethylformamid wird während 20 Stunden bei 50° gehalten. Zur Aufarbeitung wird das Lösungsmittel im Hochvakuum bei ca. 30° abgedampft und der Rückstand mit 15 ml Äthylacetat verrieben. Der ausgefallene Dicyclohexylharnstoff wird durch Abfiltrieren beseitigt, das Filtrat mit Äthylacetat auf 100 ml verdünnt, dreimal mit je 20 ml 1 N wässeriger Oxalsäure und dann mit Wasser bis zur Neutralität gewaschen, über Natriumsulfat getrocknet und im Vakuum eingedampft. Das Rohprodukt wird zur Reinigung von 30 g Kieselgel (Säule in Chloroform-Methanol 95 : 5 zubereitet) chromatographiert. Nach einem Vorlauf von 100 ml eluieren die folgenden 250 ml Chloroform-Methanol (95 : 5) das Produkt in dünnschichtchromatographisch einheitlicher Form.

DC: System (Chloroform-Methanol 9 : 1): $R_f$ 0,45.

Beispiel 4

H-Pro

Lys-Phe-Phe-(D-Trp)-Lys-Thr-Phe-Gaba

436 mg geschütztes Octapeptid der Formel

Boc-Pro

Lys-Phe-Phe-(D-Trp)-Lys(Boc)-Thr(But)-Phe-Gaba

wird bei 5° unter $N_2$ in 4 ml eines Gemisches aus 89 Vol.-% Trifluoressigsäure, 10 Vol.-% Wasser und

1 Vol.-% Thioglykolsäure gelöst, die Lösung sofort auf 25° erwärmt und nach 90 Minuten bei Raumtemperatur unter $N_2$ mit 40 ml Äther ausgefällt. Das resultierende rohe Trifluoracetat des Endprodukts wird mit Vakuum getrocknet, in 5 ml 1 N-Essigsäure gelöst und durch 15 ml Anionenaustauscher, z. B. AG® 1-X8 (ein Produkt von Bio-Rad Laboratories, Richmond, Calif., USA), in Acetatform filtriert. Das Eluat wird im Vakuum eingedampft und der Rückstand der Gegenstromverteilung im System tert-Amylalkohol-Essigsäure-Wasser-Toluol (4 : 2 : 5 : 0,8) über 260 Stufen unterzogen. Die in den Elementen 66 bis 97 enthaltenen Phasen (K = 0,45) werden gesammelt, im Vakuum eingedampft und aus tert-Butanol-Wasser (1 : 1) lyophilisiert.

Die erhaltene Titelverbindung ist in zwei Systemen dünnschichtchromatographisch einheitlich.

DC: System 157: $R_f$ 0,95; 157A: $R_f$ 0,20.

Der peptidische Ausgangsstoff ist folgendermaßen erhältlich:

### Stufe 4.1

Boc-Pro
Z-Lys-Phe-Phe-OTmse

540 mg Boc-Pro-OH, 1,78 g Z-Lys(HCl)-Phe-Phe-OTmse, 620 mg DCCI und 385 mg 1-Hydroxybenzotriazol werden in 10 ml Dimethylformamid aufgenommen, 0,319 ml N-Äthylmorpholin zugegeben und 24 Stunden bei 20° stehengelassen. Man filtriert ab, engt das Filtrat auf ca. 2 ml ein und fällt mit 50 ml Wasser aus. Nach dem Trocknen des Niederschlages über Phosphorpentoxid wird das Produkt durch Umfällen aus Äthylacetat-Petroläther gereinigt.

DC: System (Toluol-Aceton 7 : 3): $R_f$ 0,25.

### Stufe 4.2

Boc-Pro
Z-Lys-Phe-Phe-OH

960 mg

Boc-Pro
Z-Lys-Phe-Phe-OTmse

und 1,02 g Tetraäthylammoniumfluorid werden in 10 ml Dimethylformamid 45 Minuten stehengelassen. Man gibt dann 1,1 ml 1 N wässerige Salzsäure und 100 ml Wasser zu, filtriert ab, wäscht den Rückstand mit Wasser und trocknet über Phosphorpentoxid.

DC: System 157A: $R_f$ 0,30.

### Stufe 4.3

Boc-Pro
Z-Lys-Phe-Phe-(D-Trp)-Lys(Boc)-Thr(But)-Phe-OTmse

Eine Lösung von 810 mg

Boc-Pro
Z-Lys-Phe-Phe-OH

(Stufe 4.2) und 921 mg H-(D-Trp)-Lys(Boc)-Thr(But)-Phe-OTmse in 20 ml Dimethylformamid wird mit 168 mg 1-Hydroxy-benzotriazol und 250 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 5 ml Methanol verrieben und abgesaugt. Das ungelöste Material wird zur Reinigung nochmals mit 5 ml Methanol bei 50° verrieben, abgesaugt, mit Methanol gewaschen und im Vakuum getrocknet. Das Produkt ist gemäß DC einheitlich.

DC: [Chloroform-Methanol (9 : 1)] $R_f$ 0,45.

Stufe 4.4

Boc-Pro—┐

Z-Lys-Phe-Phe-(D-Trp)-Lys(Boc)-Thr(But)-Phe-OH

1,49 g

Boc-Pro—┐

Z-Lys-Phe-Phe-(D-Trp)-Lys(Boc)-Thr(But)-Phe-OTmse

(Stufe 4.3) wird in 45 ml einer frisch hergestellten, wasserfreien 0,15 M-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 0,94 ml 1 N wässeriger Salzsäure versetzt und das Produkt durch Zugabe von 200 ml Wasser gefällt. Das abfiltrierte Material wird mit Wasser gewaschen, im Vakuum über Phosphorpentoxid getrocknet und direkt weiter verwendet.

DC: System 157 B: $R_f$ 0,45.

Stufe 4.5

Boc-Pro—┐

Z-Lys-Phe-Phe-(D-Trp)-Lys(Boc)-Thr(But)-Phe-Gaba-OBzl

Ein Gemisch von 1,33 g

Boc-Pro—┐

Z-Lys-Phe-Phe-(D-Trp)-Lys(Boc)-Thr(But)-Phe-OH

(Stufe 4.4) und 425 mg Gaba-benzylester-p-toluolsulfonat in 10 ml Dimethylformamid wird mit 164 mg 1-Hydroxybenzotriazol, 22 mg DCCI und 0,146 ml N-Äthylmorpholin versetzt und 20 Stunden bei Raumtemperatur belassen. Zur Aufarbeitung wird das Gemisch mit 50 ml eiskaltem Methanol versetzt und abfiltriert. Der gewonnene Feststoff wird zur weiteren Reinigung mit 15 ml warmem Methanol während 10 Minuten verrührt, die Suspension auf 0° abgekühlt, das reine Produkt abfiltriert und im Vakuum getrocknet.

DC: [Chloroform-Methanol (9:1)] $R_f$ 0,60.

Stufe 4.6

Boc-Pro—┐

H-Lys-Phe-Phe-(D-Trp)-Lys(Boc)-Thr(But)-Phe-Gaba-OH

Ein Lösung von 1,26 g

Boc-Pro—┐

Z-Lys-Phe-Phe-(D-Trp)-Lys(Boc)-Thr(But)-Phe-Gaba-OBzl

(Stufe 4.5) in 100 ml Methanol wird nach Zugabe von 300 mg Palladium-Kohle (10%) während 6 Stunden bei Raumtemperatur und Normaldruck hydriert. Zur Aufarbeitung wird die Lösung nach Abfiltrieren des Katalysators eingedampft und das Produkt mit 25 ml peroxidfreiem Äther ausgefällt, verrieben und im Vakuum getrocknet. Das Rohprodukt wird ohne weitere Reinigung der nächsten Stufe unterzogen.

DC: System 157 B: $R_f$ 0,25.

Stufe 4.7

Boc-Pro

Lys-Phe-Phe-(D-Trp)-Lys(Boc)-Thr(But)-Phe-Gaba

Eine Lösung von 1,09 g rohem

Boc-Pro

H-Lys-Phe-Phe-(D-Trp)-Lys(Boc)-Thr(But)-Phe-Gaba-OH

(Stufe 4.6), 1,16 g 1-Hydroxybenzotriazol und 1,56 g DCCl in 760 ml Dimethylformamid wird während 20 Stunden bei 50° gehalten. Zur Aufarbeitung wird das Lösungsmittel im Hochvakuum bei ca. 30° abgedampft und der Rückstand mit 40 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 20 ml 1 N wässeriger Oxalsäure und dann mit Wasser bis zur Neutralität gewaschen, über Natriumsulfat getrocknet und im Vakuum eingedampft. Das Rohprodukt wird zur Reinigung an einer Säule von 30 g Kieselgel (zubereitet in Chloroform) chromatographiert. Nach einem Vorlauf von 300 ml Chloroform eluieren 150 ml Chloroform-Methanol (95 : 5) die Substanz in dünnschichtchromatographisch einheitlicher Form.

DC: System (Chloroform-Methanol 93 : 7): $R_f$ 0,27.

Beispiel 5

H-Phe

Lys-Phe-Phe-(D-Trp)-Lys-Thr-Phe-Gaba

665 mg geschütztes Octapeptid der Formel

Boc-Phe

Lys-Phe-Phe-(D-Trp)-Lys(Boc)-Thr(But)-Phe-Gaba

wird bei 5° unter $N_2$ in 5 ml eines Gemisches aus 89 Vol.-% Trifluoressigsäure, 10 Vol.-% Wasser und 1 Vol.-% Thioglykolsäure gelöst, die Lösung sofort auf 25° erwärmt und nach 90 Minuten bei Raumtemperatur unter $N_2$ mit 30 ml Äther ausgefällt. Das resultierende rohe Trifluoracetat des Endprodukts wird im Vakuum getrocknet, in 5 ml 1 N-Essigsäure gelöst und durch 15 ml Anionenaustauscher, z. B. AG® 1-X8 (ein Produkt von Bio-Rad Laboratories, Richmond, Calif., USA), in Acetatform filtriert. Das Eluat wird im Vakuum eingedampft und der Rückstand der Gegenstromverteilung im System tert-Amylalkohol-Essigsäure-Wasser-Toluol (4 : 2 : 5 : 0,8) über 750 Stufen unterzogen. Die in den Elementen 430 bis 470 enthaltenen Phasen (K = 1,5) werden gesammelt, im Vakuum eingedampft und aus tert-Butanol-Wasser (1 : 1) lyophilisiert.

Die erhaltene Titelverbindung ist in zwei Systemen dünnschichtchromatographisch einheitlich.

DC: System 157: $R_f$ 0,47; 157 C: $R_f$ 0,2.

Der peptidische Ausgangsstoff ist folgendermaßen erhältlich:

Stufe 5.1

Boc-Phe

Z-Lys-Phe-Phe-OTmse

2,65 g Boc-Phe-OH, 7,11 g Z-Lys(HCl)-Phe-Phe-OTmse, 2,47 g DCCl und 1,53 g 1-Hydroxybenzotriazol werden in 100 ml Dimethylformamid aufgenommen, 1,275 ml N-Äthylmorpholin zugegeben und 24 Stunden bei 20° stehengelassen. Man filtriert ab, engt das Filtrat auf ca. 5 ml ein und fällt mit 200 ml Wasser aus. Nach dem Trocknen des Niederschlages über Phosphorpentoxid wird das Produkt durch Umfällen aus Äthylacetat-Petroläther gereinigt.

DC: System 157 A: $R_f$ 0,75.

Stufe 5.2

Boc-Phe—┐
Z-Lys-Phe-Phe-OH

1,82 g

Boc-Phe—┐
Z-Lys-Phe-Phe-OTmse

(Stufe 5.1) und 1,82 g Tetraäthylammoniumfluorid werden in 20 ml Dimethylformamid 45 Minuten stehengelassen. Man gibt dann 1,96 ml 1 N wässerige Salzsäure und 200 ml Wasser zu, filtriert ab, wäscht den Rückstand mit Wasser und trocknet über Phosphorpentoxid.

DC: System 167A: $R_f$ 0,30.

Stufe 5.3

Boc-Phe—┐
Z-Lys-Phe-Phe-(D-Trp)-Lys(Boc)-Thr(But)-Phe-OTmse

Eine Lösung von 1,23 g

Boc-Phe—┐
Z-Lys-Phe-Phe-OH

(Stufe 5.2) und 1,26 g H-(D-Trp)-Lys(Boc)-Thr(But)-Phe-OTmse in 20 ml Dimethylformamid wird mit 230 mg 1-Hydroxybenzotriazol und 340 mg DCCl versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 10 ml Methanol verrieben und abgesaugt. Das ungelöste Material wird zur Reinigung nochmals mit 10 ml Methanol bei 50° verrieben, abgesaugt, mit Methanol gewaschen und im Vakuum getrocknet. Das Produkt ist gemäß DC einheitlich.

DC: [Chloroform-Methanol (93:7)] $R_f$ 0,45.

Stufe 5.4

Boc-Phe—┐
Z-Lys-Phe-Phe-(D-Trp)-Lys(Boc)-Thr(But)-Phe-OH

1,8 g

Boc-Phe—┐
Z-Lys-Phe-Phe-(D-Trp)-Lys(Boc)-Thr(But)-Phe-OTmse

(Stufe 5.3) wird in 50 ml einer frisch hergestellten, wasserfreien 0,15 M-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 1,1 ml 1 N wässeriger Salzsäure versetzt und das Produkt durch Zugabe von 200 ml Wasser gefällt. Das abfiltrierte Material wird mit Wasser gewaschen, im Vakuum über Phosphorpentoxid getrocknet und direkt weiter verwendet.

DC: System 157A: $R_f$ 0,35.

Stufe 5.5

Boc-Phe—┐
Z-Lys-Phe-Phe-(D-Trp)-Lys(Boc)-Thr(But)-Phe-Gaba-OBzl

Ein Gemisch von 1,54 g

Boc-Phe—┐
Z-Lys-Phe-Phe-(D-Trp)-Lys(Boc)-Thr(But)-Phe-OH

(Stufe 5.4) und 476 mg Gaba-benzylester-p-toluolsulfonat in 10 ml Dimethylformamid wird mit 183 mg 1-Hydroxybenzotriazol, 247 mg DCCI und 0,164 ml N-Äthylmorpholin versetzt und 20 Stunden bei Raumtemperatur belassen. Zur Aufarbeitung wird das Gemisch mit 50 ml eiskaltem Methanol versetzt und abfiltriert. Der gewonnene Feststoff wird zur weiteren Reinigung mit 5 ml warmem Methanol während 10 Minuten verrührt, die Suspension auf 0° abgekühlt, das reine Produkt abfiltriert und im Vakuum getrocknet.

DC: [Chloroform-Methanol (93 : 7)] $R_f$ 0,50.

Stufe 5.6

Boc-Phe—┐
H-Lys-Phe-Phe-(D-Trp)-Lys(Boc)-Thr(But)-Phe-Gaba-OH

Eine Lösung von 1,2 g

Boc-Phe—┐
Z-Lys-Phe-Phe-(D-Trp)-Lys(Boc)-Thr(But)-Phe-Gaba-OBzl

(Stufe 5.5) in 60 ml Dimethylformamid wird nach Zugabe von 150 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 25 ml peroxidfreiem Äther ausgefällt, abfiltriert und im Vakuum getrocknet. Das Rohprodukt wird ohne weitere Reinigung der nächsten Stufe unterzogen.

DC: System 157 B: $R_f$ 0,30.

Stufe 5.7

Boc-Phe—┐
┌—Lys-Phe-Phe-(D-Trp)-Lys(Boc)-Thr(But)-Phe-Gaba—┐
└————————————————————————┘

Eine Lösung von 1,12 g

Boc-Phe—┐
H-Lys-Phe-Phe-(D-Trp)-Lys(Boc)-Thr(But)-Phe-Gaba-OH

(Stufe 5.6), 1,07 g 1-Hydroxybenzotriazol und 1,44 g DCCI in 700 ml Dimethylformamid wird während 20 Stunden bei 50° gehalten. Zur Aufarbeitung wird das Lösungsmittel im Hochvakuum bei ca. 30° abgedampft und der Rückstand mit 30 ml Äthylacetat verrieben. Der ausgefallene Dicyclohexylharnstoff wird durch Abfiltrieren beseitigt, das Filtrat mit Äthylacetat auf 200 ml verdünnt, dreimal mit je 20 ml 1 N wässeriger Oxalsäure und dann mit Wasser bis zur Neutralität gewaschen, über Natriumsulfat getrocknet und im Vakuum eingedampft. Das Rohprodukt wird zur Reinigung an einer Säule von 30 g Kieselgel (zubereitet in Äthylacetat) chromatographiert. Nach einem Vorlauf von 100 ml Äthylacetat eluieren 250 ml Äthylacetat-Methanol (95 : 5) das Produkt in dünnschichtchromatographisch einheitlicher Form.

DC: System 157 A: $R_f$ 0,52.

In den nachstehenden Beispielen 6—11 wird die Herstellung pharmazeutischer Applikationsformen

illustriert. Die Bezeichnung »Wirkstoff« bezieht sich auf die erfindungsgemäß erhältlichen Endstoffe der Formel I, insbesondere auf diejenigen von Beispielen 1—5 und in erster Linie auf das [$Lys^5$,D-$Trp^8$, $Gaba^{12}$]-cyclo-somatostatin(5—12)octapeptid vom Beispiel 1, [$N^{\varepsilon}$-(H-Lys-Lys-Lys)-$Lys^5$,D-$Trp^8$,$Gaba^{12}$]-cyclo-somatostatin(5—12)octapeptid vom Beispiel 3 und [$N^{\varepsilon}$-(H-Pro)-$Lys^5$,D-$Trp^8$,$Gaba^{12}$]-cyclo-somatostatin(5—12)octapeptid vom Beispiel 4.

Beispiel 6

A) Eine Injektionslösung enthaltend 2,0 mg Wirkstoff wird folgendermaßen erhalten:
Man löst 1,0 mg Eisessig, 0,8 mg Natriumacetat, 8,0 mg Natriumchlorid und 2,0 mg Wirkstoff in 0,7 ml destilliertem Wasser und füllt mit destilliertem Wasser auf 1 ml auf. Die Lösung wird bei 120°C 20 Minuten lang im Autoklav erhitzt. Das pH nach der Sterilisation ist 4,5.

B) Eine Injektionslösung enthaltend 0,5 mg des Wirkstoffes wird folgendermaßen erhalten:
Man löst in 0,7 ml physiologischer Natriumchloridlösung 0,5 mg Wirkstoff und säuert die Lösung mit 0,1-N-Salzsäure auf pH 4,0 an. Mit destilliertem Wasser wird auf 1 ml aufgefüllt und sterilfiltriert.

C) Präparat enthaltend 0,5 mg Wirkstoff als sterile Trockensubstanz zur Injektion wird folgendermaßen erhalten:
Man löst 0,5 mg Wirkstoff in 1 ml einer wäßrigen Lösung von 20 mg Mannit. Die Lösung wird sterilfiltriert und unter aseptischen Bedingungen in eine 2-ml-Ampulle gefüllt, tiefgekühlt und lyophilisiert. Vor dem Gebrauch wird das Lyophilisat in destilliertem Wasser gelöst. Die Lösung wird intramuskulär oder intravenös angewendet.

Beispiel 7

Injektionspräparat enthaltend 1,0 mg Wirkstoff als Polyphosphat-Suspension wird folgendermaßen erhalten:

Man mischt eine Lösung von 1,0 mg Wirkstoff und 9,0 mg Natriumchlorid in 0,5 ml destilliertem Wasser mit einer Lösung von 2 mg Natriumpolyphosphat (Calgon N®) in 0,5 ml destilliertem Wasser. Die erhaltene Suspension hat folgende Zusammensetzung:

| | |
|---|---|
| Wirkstoff | 1,0 mg |
| Natriumpolyphosphat (Calgon N®) | 2,0 mg |
| Natriumchlorid | 9,0 mg |
| dest. Wasser bis zu | 1,0 ml |

Die Suspension weist ein pH von 6,9 auf. Sie ist für intramuskuläre Anwendung geeignet.

Beispiel 8

Injektionspräparat enthaltend 0,5 mg Wirkstoff als eine Depot-Suspension mit Dextransulfat

Man löst in 0,4 ml destilliertem Wasser 0,36 mg Essigsäure, 1,9 mg Natriumacetat-trihydrat, 8,0 mg Natriumchlorid und 0,5 mg Wirkstoff und füllt mit destilliertem Wasser auf 0,5 ml auf. Zu dieser Lösung wird unter Rühren 0,5 ml einer 0,1 %igen Lösung von Dextransulfat (Molekulargewicht 500 000) gegeben, womit sich eine homogene Ausfällung bildet. Die erhaltene Suspension hat folgende Zusammensetzung:

| | |
|---|---|
| Wirkstoff | 0,50 mg |
| Dextransulfat MG 500 000 | 0,50 mg |
| Essigsäure 100% | 0,36 mg |
| Natriumacetat-trihydrat | 1,90 mg |
| Natriumchlorid | 8,00 mg |
| dest. Wasser bis zu | 1,00 ml |

Die wäßrige Suspension ist für intramuskuläre und subcutane Injektion geeignet.

Beispiel 9

Nasal-Spray

30 mg feingemahlener Wirkstoff wird in einer Mischung von 75 mg Benzylalkohol und 1,395 g eines Gemisches von halbsynthetischen Glyceriden gesättigter Fettsäuren mit 8—12 Kohlenstoffatomen (z. B. Miglyol® 812) suspendiert. Mit dieser Suspension werden Aluminium-Monoblocdosen (Gehalt 10 ml ) eingefüllt, mit einem Dosierventil verschlossen und mit 6,0 g eines Gemisches von Dichlordifluormethan und 1,2-Dichlor-1,1,2,2-tetrafluoräthan (z. B. Freon® 12/114) im Vol.-Verhältnis 40 : 60 unter Stickstoffdruck eingefüllt. Die Aluminiumdose mit einem Füllgewicht von insgesamt 7,5 g enthält 100 Einzeldosen à 0,3 mg Wirkstoff. Die Sprühdose ist mittels des Ventils so eingestellt, daß bei einmaligem Druck eine Einzeldose versprüht wird.

In gleicher Weise werden Nasensprays hergestellt, welche statt Miglyol® die gleiche Gewichtsmenge Isopropylmyristat oder Isopropylpalmitat oder einer Mischung von Glycerin- und Polyoxyäthylenglykolestern von Fettsäuren mit 8 und 10 Kohlenstoffatomen (z. B. Labrafac® WL 1219) enthalten.

Beispiel 10

Manteltabletten oder Dragées enthaltend ca. 45 mg Wirkstoff
für kombinierte rasche und langsame Freigabe

Unter Anwendung üblicher Mischverfahren werden homogene Mischungen folgender Zusammensetzung zubereitet (Menge für 10000 Stück)

Mischung A:

| | |
|---|---|
| Wirkstoff | 200 g |
| Lactose, wasserfrei, für Direkttablettierung | 940 g |
| Cellulose, mikrokristallin | 650 g |
| Magnesiumstearat | 10 g |

Mischung B:

| | |
|---|---|
| Wirkstoff | 250 g |
| Lactose, mikrokristallin, für Direkttablettierung | 400 g |
| hydriertes Rizinusöl, feinkörnig | 50 g |

Aus den Mischungen A und B werden Manteltabletten gepreßt: Zuerst wird aus der Mischung B der Kern mit verlängerter Wirkung, der ein Gewicht von 70 mg und einen Durchmesser von 6,5 mm aufweist, gepreßt. Zur Herstellung des Mantels mit rascher Freigabe des Wirkstoffes wird Mischung A in einer Gewichtsmenge von 180 mg genommen, und es werden schwach gewölbte Stempel mit einem Durchmesser von 8 mm verwendet, so daß Manteltabletten von einem Gesamtgewicht von 250 mg und einem Gehalt von 20 mg des Wirkstoffes in einer rasch wirkenden Form und 25 mg in der Retard-Form resultieren.

Die erhaltenen Manteltabletten werden, wenn gewünscht, in Chargen von 100000 Stück in einem Dragierkessel von 65 cm Durchmesser mittels einer automatischen Sprühanlage mit einer herkömmlichen Lacklösung in der üblichen Zusammensetzung lackiert.

Die Temperatur der Zuluft beträgt 60°C, die Temperatur der Kerne wird bei etwa 25°C gehalten. Anschließend werden die fertigen Dragées während 12 Stunden bei 30°C nachgetrocknet.

Beispiel 11

Steckkapseln enthaltend ca. 100 mg Wirkstoff

Eine Mischung der folgenden Zusammensetzung (für 10000 Stück) wird zubereitet:

| | |
|---|---|
| Wirkstoff | 1000 g |
| Maisstärke | 300 g |
| Lactose, kristallin, mittlere Teilchengröße von etwa 125 μm | 400 g |
| Lactose, feingemahlen | 200 g |
| Calciumstearat | 50 g |

Diese Mischung wird nach Bedarf noch zerrieben und homogenisiert. Das Pulver wird gesiebt und in Portionen zu je 195 mg in Gelatine-Steckkapseln trocken abgefüllt. Die fertigen Kapseln enthalten je ca. 100 mg Wirkstoff.

**Patentansprüche für die Vertragsstaaten: BE, CH, DE, FR, IT, LI, LU, NL, SE**

1. Verfahren zur Herstellung eines cyclischen Octapeptids der Formel

Aaa($Ac_A$)-Phe-Phe-trp-Lys($Ac_B$)-Thr-Phe-Gaba (I)

5 6 7 8 9 10 11 12

worin

Aaa der Rest einer geradkettigen $\alpha,\omega$-Diaminoalkansäure mit 4—7 C-Atomen,
trp der Rest L-Trp oder D-Trp, oder ein davon abgeleiteter Rest, der im Indolkern ein Halogen trägt,
$Ac_A$ ein an der $\omega$-Aminogruppe befindlicher Acylrest Ac einer gegebenenfalls substituierten Carbonsäure, die Amidinogruppe oder Wasserstoff, und
$Ac_B$ ein an der $\varepsilon$-Aminogruppe befindlicher Acylrest $Ac^1$ einer Aminosäure oder eines Oligopeptids, oder Wasserstoff

ist, sowie von Salzen und Komplexen davon, dadurch gekennzeichnet, daß man in einer strukturell entsprechenden Verbindung, in welcher mindestens eine von vorhandenen Amino-, Hydroxyl- und/oder Carboxylgruppen eine Schutzgruppe $X_o$, $X_a$, Y, bzw. W trägt, vorhandene Schutzgruppen abspaltet und, wenn erwünscht, in einer erhaltenen Verbindung mit freier $\omega$-Aminogruppe des $Aaa^5$ und/oder des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

2. Ein cyclisches Octapeptid der Formel

Aaa($Ac_A$)-Phe-Phe-trp-Lys($Ac_B$)-Thr-Phe-Gaba (I)

5 6 7 8 9 10 11 12

worin

Aaa der Rest einer geradkettigen $\alpha,\omega$-Diaminoalkansäure mit 4—7 C-Atomen,
trp der Rest L-Trp oder D-Trp, oder ein davon abgeleiteter Rest, der im Indolkern ein Halogen trägt,
$Ac_A$ ein an der $\omega$-Aminogruppe befindlicher Acylrest Ac einer gegebenenfalls substituierten Carbonsäure, die Amidinogruppe oder Wasserstoff, und
$Ac_B$ ein an der $\varepsilon$-Aminogruppe befindlicher Acylrest $Ac^1$ einer Aminosäure oder eines Oligopeptids, oder Wasserstoff

ist, oder ein Salz oder ein Komplex davon.

3. Ein cyclisches Octapeptid gemäß Anspruch 2, worin in der Formel I trp für D-Trp, Aaa für Lys, Orn oder Arg, $Ac_A$ für Wasserstoff oder den Acylrest einer in der Natur vorkommenden $\alpha$-Aminosäure oder eines entsprechenden Oligopeptids und $Ac_B$ für Wasserstoff steht, sowie physiologisch verträgliche Salze und therapeutisch verwendbare Komplexe davon.

4. Ein cyclisches Peptid gemäß Anspruch 2, welches das [$Lys^5$,D-$Trp^8$,$Gaba^{12}$]-cyclo-somatostatin(5—12)octapeptid oder ein physiologisch verträgliches Salz oder ein therapeutisch verwendbarer Komplex davon ist.

5. Ein cyclisches Peptid gemäß Anspruch 2, welches das [$Orn^5$,D-$Trp^8$,$Gaba^{12}$]-cyclo-somatostatin(5—12)octapeptid oder ein physiologisch verträgliches Salz oder ein therapeutisch verwendbarer Komplex davon ist.

6. Ein cyclisches Peptid gemäß Anspruch 2, welches das [$N^{\varepsilon}$-(Pro)-$Lys^5$,D-$Trp^8$,$Gaba^{12}$]-cyclo-somatostatin(5—12)-octapeptid oder ein physiologisch verträgliches Salz oder ein therapeutisch verwendbarer Komplex davon ist.

7. Ein cyclisches Peptid gemäß Anspruch 2, welches das [$N^{\varepsilon}$-(Phe)-$Lys^5$,D-$Trp^8$,$Gaba^{12}$]-cyclo-somatostatin(5—12)octapeptid oder ein physiologisch verträgliches Salz oder ein therapeutisch verwendbarer Komplex davon ist.

8. Ein cyclisches Peptid gemäß Anspruch 2, welches das [$N^{\varepsilon}$-(Lys-Lys-Lys)-$Lys^5$,D-$Trp^8$,$Gaba^{12}$]-cyclo-somatostatin(5—12)-octapeptid oder ein physiologisch verträgliches Salz oder ein therapeutisch verwendbarer Komplex davon ist.

9. Eine Verbindung gemäß einem der Ansprüche 2 bis 8 als Antidiabeticum und/oder ein Mittel zur Kontrolle von gastro-intestinalen Blutungen.

10. Eine pharmazeutische Zusammensetzung enthaltend mindestens eine Verbindung gemäß einem der Ansprüche 2 bis 9.

**Patentansprüche für den Vertragsstaat: AT**

1. Verfahren zur Herstellung eines cyclischen Octapeptids der Formel

$$\overline{\text{Aaa(Ac}_A\text{)-Phe-Phe-trp-Lys(Ac}_B\text{)-Thr-Phe-Gaba}} \qquad \text{(I)}$$

5 6 7 8 9 10 11 12

worin

Aaa der Rest einer geradkettigen $\alpha,\omega$-Diaminoalkansäure mit 4—7 C-Atomen,
trp der Rest L-Trp oder D-Trp, oder ein davon abgeleiteter Rest, der im Indolkern ein Halogen trägt,
$Ac_A$ ein an der $\omega$-Aminogruppe befindlicher Acylrest Ac einer gegebenenfalls substituierten Carbonsäure, die Amidinogruppe oder Wasserstoff, und
$Ac_B$ ein an der $\varepsilon$-Aminogruppe befindlicher Acylrest $Ac^1$ einer Aminosäure oder eines Oligopeptids, oder Wasserstoff

ist, sowie von Salzen und Komplexen davon, dadurch gekennzeichnet, daß man in einer strukturell entsprechenden Verbindung, in welcher mindestens eine von vorhandenen Amino-, Hydroxyl- und/oder Carboxylgruppen eine Schutzgruppe $X_o$, $X_a$, Y bzw. W trägt, vorhandene Schutzgruppen abspaltet und, wenn erwünscht, in einer erhaltenen Verbindung mit freier $\omega$-Aminogruppe des $Aaa^5$ und/oder des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

2. Verfahren gemäß Anspruch 1 zur Herstellung eines cyclischen Octapeptids der im Anspruch 1 definierten Formel I, dadurch gekennzeichnet, daß man die nachträgliche fakultative Acylierung in der Weise durchführt, daß man ein erhaltenes Cyclopeptid der Formel

$$\overline{\text{Aaa(Ac}_A\text{)-Phe-Phe-trp-Lys(Ac}_B\text{)-Thr(Y}_o\text{)-Phe-Gaba}} \qquad \text{(III)}$$

worin $Y_o$ Wasserstoff oder eine Hydroxylschutzgruppe Y ist und trp, Aaa, $Ac_A$ und $Ac_B$ die im Anspruch 1 definierte Bedeutung mit der Maßgabe haben, daß mindestens einer der Reste $Ac_A$ und $Ac_B$ Wasserstoff ist und im anderen vorhandene Amino- und/oder Hydroxylgruppen in vorübergehend geschützter Form vorliegen, mit einer Carbonsäure $Ac_0OH$, worin $Ac_0$ einen den im Anspruch 1 definierten Resten Ac bzw. $Ac^1$ entsprechenden Rest bedeutet, in welchem vorhandene Amino- und Hydroxylgruppen Schutzgruppen $X_o$, $X_a$ bzw. Y tragen können oder mit einem reaktionsfähigen Derivat einer solchen Säure behandelt und, wenn erwünscht oder notwendig ist, im erhaltenen Produkt durch Abspaltung der Schutzgruppen $X_o$, $X_a$ und Y Aminogruppen bzw. Hydroxylgruppen freisetzt.

3. Verfahren gemäß Anspruch 1, dadurch gekennzeichnet, daß man ein lineares Peptid der Formel

$$H—[I_a]—V \qquad \text{(II)}$$

worin $I_a$ einen der Formel I entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, wobei übrige vorhandene, an der Cyclisierungsreaktion nicht beteiligte Amino-, Carboxyl- und Hydroxylgruppen nach Bedarf in geschützter Form vorliegen und anschließend freigesetzt werden, cyclisiert und im erhaltenen Cyclopeptid, in welchem mindestens eine von vorhandenen Amino-, Hydroxyl- und/oder Carboxylgruppe eine Schutzgruppe $X_o$, $X_a$, Y bzw. W trägt, vorhandene Schutzgruppen, gewünschtenfalls nach Acylierung einer vorhandenen freien oder freigesetzten $\omega$-Aminogruppe im Rest $Aaa^5$ und/oder $Lys^9$, vorhandene Schutzgruppen abspaltet.

4. Verfahren gemäß einem der Ansprüche 1—3, dadurch gekennzeichnet, daß man ein cyclisches Octapeptid der Formel I, worin trp für D-Trp, Aaa für Lys, Orn oder Arg, $Ac_A$ für Wasserstoff oder den Acylrest einer in der Natur vorkommenden $\alpha$-Aminosäure oder eines entsprechenden Oligopeptids und $Ac_B$ für Wasserstoff steht, oder ein physiologisch verträgliches Salz oder einen therapeutisch verwendbaren Komplex davon herstellt.

5. Verfahren gemäß Anspruch 1 oder 3, dadurch gekennzeichnet, daß man [$Lys^5$, D-$Trp^8$, $Gaba^{12}$]-cyclo-somatostatin(5—12)octapeptid oder ein physiologisch verträgliches Salz oder einen therapeutisch verwendbaren Komplex davon herstellt.

6. Verfahren gemäß Anspruch 1 oder 3, dadurch gekennzeichnet, daß man [$Orn^5$, D-$Trp^8$, $Gaba^{12}$]-cyclo-somatostatin(5—12)octapeptid oder ein physiologisch verträgliches Salz oder einen therapeutisch verwendbaren Komplex davon herstellt.

7. Verfahren gemäß einem der Ansprüche 1—3, dadurch gekennzeichnet, daß man [$N^\varepsilon$-(Pro)-$Lys^5$, D-$Trp^8$, $Gaba^{12}$]-cyclo-somatostatin(5—12)-octapeptid oder ein physiologisch verträgliches Salz oder einen therapeutisch verwendbaren Komplex davon herstellt.

8. Verfahren gemäß einem der Ansprüche 1—3, dadurch gekennzeichnet, daß man [$N^\varepsilon$-(Phe)-$Lys^5$, D-$Trp^8$, $Gaba^{12}$]-cyclo-somatostatin(5—12)-octapeptid oder ein physiologisch verträgliches Salz oder einen therapeutisch verwendbaren Komplex davon herstellt.

9. Verfahren gemäß einem der Ansprüche 1—3, dadurch gekennzeichnet, daß man [$N^\varepsilon$-(Lys-Lys-Lys)-$Lys^5$, D-$Trp^8$, $Gaba^{12}$]-cyclo-somatostatin-(5—12)-octapeptid oder ein physiologisch verträgliches Salz oder einen therapeutisch verwendbaren Komplex davon herstellt.

10. Verfahren zur Herstellung, auf nicht-chemischem Wege, einer pharmazeutischen Zusammensetzung enthaltend mindestens eine gemäß einem der Ansprüche 1 bis 9 erhältliche Verbindung.

**Claims for the Contracting States: BE, CH, DE, FR, IT, LI, LU, NL, SE**

1. A process for the preparation of a cyclic octapeptide of the formula

$$\overline{\text{Aaa}(\text{Ac}_A)\text{-Phe-Phe-trp-Lys}(\text{Ac}_B)\text{-Thr-Phe-Gaba}} \quad (I)$$

5 6 7 8 9 10 11 12

wherein

Aaa is the radical of a straight chain $\alpha,\omega$-diaminoalkanoic acid containing 4 to 7 carbon atoms,
trp is a radical L-Trp or D-Trp, or a radical derived therefrom, which radical carries a halogen atom in the indole nucleus,
$Ac_A$ is an acyl radical Ac of an unsubstituted or substituted carboxylic acid, which acyl radical is positioned at the $\omega$-amino group, or $Ac_A$ is an amidino group or hydrogen, and
$Ac_B$ is an acyl radical $Ac^1$ of an amino acid or of an oligopeptide, which acyl radical is positioned at the $\varepsilon$-amino group, or $Ac_B$ is hydrogen,

or of a salt or complex thereof, which process comprises removing any protecting groups present from a structurally corresponding compound, wherein at least one of the amino, hydroxyl and/or carboxyl groups present carries a protecting group $X_o$, $X_a$, Y or W, and, if desired, acylating in a resultant compound containing a free $\omega$-amino group in the $Aaa^5$ and/or $Lys^9$ radical at said amino group, and/or, if desired, converting a resultant compound of basic character into an acid addition salt thereof, or converting such an acid addition salt into the corresponding base, or, if desired, converting a resultant compound of acidic character ito a salt thereof, or converting such a salt into the corresponding acidic compound, and/or, if desired, converting a resultant compound into its complex.

2. A cyclic octapeptide of the formula

$$\overline{\text{Aaa}(\text{Ac}_A)\text{-Phe-Phe-trp-Lys}(\text{Ac}_B)\text{-Thr-Phe-Gaba}} \quad (I)$$

5 6 7 8 9 10 11 12

wherein

Aaa is the radical of a straight chain $\alpha,\omega$-diaminoalkanoic acid containing 4 to 7 carbon atoms,
trp is a radical L-Trp or D-Trp, or a radical derived therefrom, which radical carries a halogen atom in the indole nucleus,
$Ac_A$ is an acyl radical Ac of an unsubstituted or substituted carboxylic acid, which acyl radical is positioned at the $\omega$-amino group, or $Ac_A$ is an amidino group or hydrogen, and
$Ac_B$ is an acyl radical $Ac^1$ of an amino acid or of an oligopeptide, which acyl radical is positioned at the $\varepsilon$-amino group, or $Ac_B$ is hydrogen,

or a salt or complex thereof.

3. A cyclic octapeptide according to claim 2, wherein in the formula I trp is D-Trp, Aaa is Lys, Orn or Arg, $Ac_A$ is hydrogen or the acyl radical of a naturally occurring $\alpha$-amino acid or of a corresponding oligopeptide, and $Ac_B$ is hydrogen, or a physiologically acceptable salt or a therapeutically acceptable complex thereof.

4. A cyclic peptide according to claim 2, which is [$Lys^5$,D-$Trp^8$,$Gaba^{12}$]-cyclosomatostatin(5—12)-octapeptide, or a physiologically acceptable salt or a therapeutically acceptable complex thereof.

5. A cyclic peptide according to claim 2, which is [$Orn^5$,D-$Trp^8$,$Gaba^{12}$]cyclosomatostatin(5—12)octapeptide, or a physiologically acceptable salt or a therapeutically acceptable complex thereof.

6. A cyclic peptide according to claim 2, which is [$N^{\varepsilon}$-(Pro)-$Lys^5$,D-$Trp^8$,$Gaba^{12}$]cyclosomatostatin(5—12)-octapeptide, or a physiologically acceptable salt or a therapeutically acceptable complex thereof.

7. A cyclic peptide according to claim 2, which is [$N^{\varepsilon}$-(Phe)-$Lys^5$,D-$Trp^8$,$Gaba^{12}$]cyclosomatostatin(5—12)octapeptide, or a physiologically acceptable salt or a therapeutically acceptable complex thereof.

8. A cyclic peptide according to claim 2, which is [$N^{\varepsilon}$-(Lys-Lys-Lys)-$Lys^5$,D-$Trp^9$,$Gaba^{12}$]cyclosomatostatin(5—12)octapeptide, or a physiologically salt or a therapeutically acceptable complex thereof.

9. Use of a compound according to any one of claims 2 to 8 as antidiabetic and/or agent for controlling gastro-inestinal bleeding.

10. A pharmaceutical composition containing at least one compound according to any one of claims 2 to 9.

**Claims for the Contracting State: AT**

1. A process for the preparation of a cyclic octapeptide of the formula

$$\overline{\underline{\text{Aaa}(\text{Ac}_A)\text{-Phe-Phe-trp-Lys}(\text{Ac}_B)\text{-Thr-Phe-Gaba}}} \qquad (I)$$

5 6 7 8 9 10 11 12

wherein

Aaa is the radical of a straight chain $\alpha,\omega$-diaminoalkanoic acid containing 4 to 7 carbon atoms,
trp is a radical L-Trp or D-Trp, or a radical derived therefrom, which radical carries a halogen atom in the indole nucleus,
$Ac_A$ is an acyl radical Ac of an unsubstituted or substituted carboxylic acid, which acyl radical is positioned at the $\omega$-amino group, or $Ac_A$ is an amidino group or hydrogen, and
$Ac_B$ is an acyl radical $Ac^1$ of an amino acid or of an oligopeptide, which acyl radical is positioned at the $\varepsilon$-amino group, or $Ac_B$ is hydrogen,

or of a salt or complex thereof, which process comprises removing any protecting groups present from a structurally corresponding compound, wherein at least one of the amino, hydroxyl and/or carboxyl groups present carries a protecting group $X_o$, $X_a$, Y or W, if desired, acylating in a resultant compound containing a free $\omega$-amino group in the $Aaa^5$ and/or $Lys^9$ radical at said amino group, and/or, if desired, converting a resultant compound of basic character into an acid addition salt thereof, or converting such an acid addition salt into the corresponding base, or, if desired, converting a resultant compound of acidic character into a salt thereof, or converting such a salt into the corresponding acidic compound, and/or, if desired, converting a resultant compound into its complex.

2. A process according to claim 1 for the preparation of a cyclic octapeptide of the formula I as defined in claim 1, which process comprises effecting the subsequent optional acylation by treating a resultant cyclopeptide of the formula

$$\overline{\underline{\text{Aaa}(\text{Ac}_A)\text{-Phe-Phe-trp-Lys}(\text{Ac}_B)\text{-Thr}(\text{Y}_o)\text{-Phe-Gaba}}} \qquad (III)$$

wherein $Y_o$ is hydrogen or a protecting hydroxyl group Y, and trp, Aaa, $Ac_A$ and $Ac_B$ are as defined in claim 1, with the proviso that at least one of the radicals $Ac_A$ and $Ac_B$ is hydrogen, and amino and/or hydroxyl groups present in the other radical are temporarily protected, with a carboxylic acid $Ac_0OH$, wherein $Ac_0$ is a radical corresponding to the radical Ac or $Ac^1$ as defined in claim 1, in which radical $Ac_0$ amino and hydroxyl groups present may carry protecting groups $X_o$, $X_a$ or Y, or with a reactive derivative of such an acid, and, if desired or necessary, liberating amino groups or hydroxyl groups in the resultant product by removing the protecting groups $X_o$, $X_a$ and Y.

3. A process according to claim 1, which process comprises cyclising a linear peptide of the formula

$$H - [I_a] - V \qquad (II)$$

wherein $I_a$ is a radical corresponding to the formula I, in which radical the amide bond between any two adjacent amino acid esters of the peptide ring is interrupted, and V is a free hydroxyl group, a hydroxyl group modified by an activating group, or V is the hydrazino group $-NH-NH_2$, wherein remaining amino, carboxyl and hydroxyl groups present that are not participating in the cyclisation reaction are, where necessary, in protected form and are subsequently liberated, and removing any protecting groups present from the resultant cyclopeptide, wherein at least one of the amino, hydroxyl and/or carboxyl groups present carries a protecting group $X_o$, $X_a$, Y or W, if desired after acylating a free or liberated $\omega$-amino group present in the radical $Aaa^5$ and/or $Lys^9$.

4. A process according to any one of claims 1 to 3, which process comprises preparing a cyclic octapeptide of the formula I, wherein trp is D-Trp, Aaa is Lys, Orn or Arg, $Ac_A$ is hydrogen or the acyl radical of a naturally occurring $\alpha$-amino acid or of a corresponding oligopeptide, and $Ac_B$ is hydrogen, or a physiologically acceptable salt or a therapeutically acceptable complex thereof.

5. A process according to either claim 1 or claim 3, which comprises preparing [$Lys^5$,D-$Trp^8$,$Gaba^{12}$]cyclosomatostatin(5—12)octapeptide, or a physiologically acceptable salt or a therapeutically acceptable complex thereof.

6. A process according to either claim 1 or claim 3, which comprises preparing [$Orn^5$,D-$Trp^8$,$Gaba^{12}$]cyclosomatostatin(5—12)octapeptide, or a physiologically acceptable salt or a therapeutically acceptable complex thereof.

7. A process according to any one of claims 1 to 3, which comprises preparing [$N^\varepsilon$-(Pro)-$Lys^5$,D-$Trp^8$,$Gaba^{12}$]cyclosomatostatin(5—12)octapeptide, or a physiologically acceptable salt or a therapeutically acceptable complex thereof.

8. A process according to any one of claims 1 to 3, which comprises preparing [$N^\varepsilon$-(Phe)-$Lys^5$,D-$Trp^8$,$Gaba^{12}$]cyclosomatostatin(5—12)octapeptide, or a physiologically acceptable salt or a therapeutically acceptable complex thereof.

9. A process according to any one of claims 1 to 3, which comprises preparing [$N^\varepsilon$-(Lys-Lys-Lys)-$Lys^5$,D-$Trp^8$,$Gaba^{12}$]cyclosomatostatin(5—12)octapeptide, or a physiologically acceptable salt or a therapeutically acceptable complex thereof.

10. A process for the preparation by non-chemical means of a pharmaceutical composition containing at least one of the compounds which are obtainable according to any one of claims 1 to 9.

**Revendications pour les Etats contractants: BE, CH, DE, FR, IT, LI, LU, NL, SE**

1. Procédé de préparation d'un octapeptide cyclique de formule

$$\overline{\underline{|\text{Aaa}(Ac_A)\text{-Phe-Phe-trp-Lys}(Ac_B)\text{-Thr-Phe-Gaba}|}} \qquad (I)$$

5 6 7 8 9 10 11 12

dans laquelle

Aaa représente le reste d'un acide $\alpha,\omega$-diaminoalcanoïque à chaîne droite en C 4—C 7,

trp représente le reste L-Trp ou D-Trp ou un reste dérivé de ceux-ci qui porte un halogène sur le noyau indole,

$Ac_A$ représente un radical acyle Ac d'un acide carboxylique éventuellement substitué qui se trouve sur le groupe $\omega$-amino, le groupe amidino ou l'hydrogène, et

$Ac_B$ est un radical acyle $Ac^1$ d'un aminoacide ou d'un oligopeptide placé sur le groupe $\varepsilon$-amino, ou l'hydrogène,

ainsi que des sels et complexes d'un tel composé, caractérisé en ce que, dans un composé de structure correspondante dans lequel au moins un des groupes amino, hydroxy et/ou carboxyle présents porte un groupe protecteur $X_o$, $X_a$, Y ou W respectivement, on élimine les groupes protecteurs présents et, si on le désire, dans un composé obtenu dont le groupe $\omega$-amino du reste $Aaa^5$ et/ou $Lys^9$ est libre, on acyle sur ce groupe amino, et/ou, si on le désire, on convertit un composé obtenu possédant un caractère basique en un sel par addition avec un acide ou bien on convertit un tel sel d'acide en la base correspondante, ou bien, sie on le désire, on convertit un composé obtenu possédant un caractère acide en un sel ou un tel sel en le composé acide correspondant, et/ou, si on le désire, on convertit un composé obtenu en son complexe.

2. Un octapeptide cyclique de formule

$$\overline{\text{Aaa}(\text{Ac}_A)\text{-Phe-Phe-trp-Lys}(\text{Ac}_B)\text{-Thr-Phe-Gaba}} \quad \text{(I)}$$

5 6 7 8 9 10 11 12

dans laquelle

Aaa représente le reste d'un acide $\alpha,\omega$-diaminoalcanoïque à chaîne droite en C 4—C 7,
trp représente le reste L-Trp ou D-Trp, ou un reste dérivé de ceux-ci, portant un halogène sur le noyau indole,
$Ac_A$ représente un radical acyle Ac d'un acide carboxylique éventuellement substitué placé sur le groupe $\omega$-amino, le groupe amidino ou l'hydrogène, et
$Ac_B$ représente un radical acyle $Ac^1$ d'un aminoacide ou d'un oligopeptide placé sur le groupe $\varepsilon$-amino, ou l'hydrogène,

ou un sel ou un complexe d'un tel composé.

3. Un octapeptide cyclique selon la revendication 2, dans lequel, dans la formule I, trp représente D-Trp, Aaa représente Lys, Orn ou Arg, $Ac_A$ représente l'hydrogène ou le radical acyle d'un $\alpha$-aminoacide existant dans la nature ou d'un oligopeptide correspondant, et $Ac_B$ représente l'hydrogène, ainsi que les sels acceptables pour l'usage pharmaceutique et complexes utilisables en thérapeutique de ce composé.

4. Un peptide cyclique selon la revendication 2, qui consiste en le [$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

5. Un peptide cyclique selon la revendication 2, qui consiste en le [$Orn^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

6. Un peptide cyclique selon la revendication 2, qui consiste en le [$N^\varepsilon$-(Pro)-$Lys^5$,D-$Trp^8$,$Gaba^{12}$]-cyclo-somatostatine(5—12)-octapeptide ou un sel acceptable pour l'usage pharmaceutique ou un complexe utilisable en thérapeutique de ce composé.

7. Un peptide cyclique selon la revendication 2, qui consiste en le [$N^\varepsilon$-(Phe)-$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

8. Un peptide cyclique selon la revendication 2, qui consiste en le [$N^\varepsilon$-(Lys-Lys-Lys)-$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

9. Un composé selon l'une des revendications 2 à 8, en tant qu'antidiabétique et/ou agent pour le traitement des saignements gastro-intestinaux.

10. Une composition pharmaceutique contenant au moins un composé selon l'une des revendications 2 à 9.

## Revendications pour l'Etat contractant: AT

1. Procédé de préparation d'un octapeptide cyclique de formule

$$\overline{\text{Aaa}(\text{Ac}_A)\text{-Phe-Phe-trp-Lys}(\text{Ac}_B)\text{-Thr-Phe-Gaba}} \quad \text{(I)}$$

5 6 7 8 9 10 11 12

dans laquelle

Aaa représente le reste d'un acide $\alpha,\omega$-diaminoalcanoïque à chaîne droite en C 4—C 7,
trp représente le reste L-Trp ou D-Trp ou un reste dérivé de ceux-ci portant un halogène sur le noyau indole,
$Ac_A$ représente un radical acyle Ac d'un acide carboxylique éventuellement substitué placé sur le groupe $\omega$-amino, le groupe amidino ou l'hydrogène, et
$Ac_B$ représente un radical acyle $Ac^1$ d'un aminoacide ou d'un oligopeptide placé sur le groupe $\varepsilon$-amino, ou l'hydrogène,

ainsi que de sels et complexes de ce composé, caractérisé en ce que, dans un composé à structure correspondante, dans lequel au moins un des groupes amino, hydroxy et/ou carboxyle présents porte un groupe protecteur $X_o$, $X_a$, Y ou W respectivement, on élimine les groupes protecteurs présents, si on le désire, dans un composé obtenu dans lequel le groupe $\varepsilon$-amino du reste $Aaa^5$ et/ou $Lys^9$ est libre, on acyle sur ce groupe amino, et/ou, si on le désire, on convertit un composé obtenu possédant un caractère basique en un sel par addition avec un acide, ou on convertit un tel sel d'acide en la base correspondante, ou bien, si on le désire, on convertit un composé obtenu possédant un caractère acide en un sel ou un tel sel en le composé acide correspondant, et/ou, si on le désire, on convertit un composé obtenu en son complexe.

2. Procédé selon la revendication 1, pour la préparation d'un octapeptide cyclique de formule I définie dans la revendication 1, caractérisé en ce que l'acylation facultative subséquente est effectuée en traitant un cyclopeptide obtenu de formule

$$\overline{\text{Aaa}(Ac_A)\text{-Phe-Phe-trp-Lys}(Ac_B)\text{-Thr}(Y_o)\text{-Phe-Gaba}} \qquad \text{(III)}$$

dans laquelle $Y_o$ représente l'hydrogène ou un groupe protecteur Y du groupe hydroxy et trp, Aaa, $Ac_A$ et $Ac_B$ ont les significations indiquées dans la revendication 1, étant spécifié toutefois qu'au moins un des symboles $Ac_A$ et $Ac_B$ représente l'hydrogène et que les groupes amino et/ou hydroxy présents dans le reste représenté par l'autre de ces symboles sont transitoirement à l'état protégé, à l'aide d'un acide carboxylique $Ac_0OH$ dans lequel $Ac_0$ représente un reste correspondant aux restes respectifs Ac et $Ac^1$ définis dans la revendication 1, et dans lequel les groupes amino et hydroxy présents peuvent porter des groupes protecteurs respectifs $X_o$, $X_a$ ou Y, ou à l'aide d'un dérivé réactif d'un tel acide, et, si on le désire ou si c'est nécessaire, on libère les groupes amino et hydroxy du produit obtenu par élimination des groupes protecteurs respectifs $X_o$, $X_a$ et Y.

3. Procédé selon la revendication 1, caractérisé en ce que l'on cyclise un peptide linéaire de formule

$$H—[I_a]—V \qquad \text{(II)}$$

dans laquelle $I_a$ représente un reste répondant à la formule I, dans lequel la liaison amidique entre deux restes d'aminoacides voisins quelconques du cycle peptide est interrompue, et V représente un groupe hydroxy libre, un groupe hydroxy modifié par un groupe activant ou le groupe hydrazino—NH—$NH_2$, les autres groupes amino, carboxyle et hydroxy présents, ne participant pas à la réaction de cyclisation, étant si nécessaire à l'état protégé puis libérés, et dans le cyclopeptide obtenu, dans lequel au moins un des groupes amino, hydroxy et/ou carboxyle présents porte un groupe protecteur respectif $X_o$, $X_a$, Y ou W, on élimine les groupes protecteurs présents, éventuellement après acylation d'un groupe $\omega$-amino libre ou libéré dans le reste $Aaa^5$ et/ou $Lys^9$.

4. Procédé selon l'une des revendications 1 à 3, caractérisé en ce que l'on prépare un octapeptide cyclique de formule I dans laquelle trp représente D-Trp, Aaa représente Lys, Orn ou Arg, $Ac_A$ représente l'hydrogène ou le reste acyle d'un $\alpha$-aminoacide existant dans la nature ou d'un oligopeptide correspondant, et $Ac_B$ représente l'hydrogène, ou un sel acceptable pour l'usage pharmaceutique ou un complexe utilisable en thérapeutique d'un tel composé.

5. Procédé selon la revendication 1 ou 3, caractérisé en ce que l'on prépare le [$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

6. Procédé selon la revendication 1 ou 3, caractérisé en ce que l'on prépare le [$Orn^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

7. Procédé selon l'une des revendications 1 à 3, caractérisé en ce que l'on prépare le [$N^\varepsilon$-(Pro)-$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

8. Procédé selon l'une des revendications 1 à 3, caractérisé en ce que l'on prépare le [$N^\varepsilon$-(Phe)-$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

9. Procédé selon l'une des revendications 1 à 3, caractérisé en ce que l'on prépare le [$N^\varepsilon$(Lys-Lys-Lys)-$Lys^5$,D-$Trp^8$,$Gaba^{12}$]-cyclo-somatostatine(5—12)octapeptide ou un sel acceptable pour l'usage pharmaceutique ou un complexe utilisable en thérapeutique de ce composé.

10. Procédé de préparation par voie non chimique d'une composition pharmaceutique contenant au moins un composé susceptible d'être obtenu selon l'une des revendications 1 à 9.